# EUROPEAN PATENT APPLICATION

(11) **EP 3 372 613 A1**
(43) Date of publication of application: **12.09.2018**
(21) Application number: 16862456.7
(22) Date of filing: 03.11.2016
(51) Int. Cl.: C07K 7/08, A61K 38/10

(54) **PEPTIDE HAVING NEURONAL LOSS PREVENTION AND REGENERATION EFFECTS, AND COMPOSITION CONTAINING SAME**

(30) Priority: 03.11.2015 KR 20150153518
(71) Applicant: Gemvax & Kael Co., Ltd., Daejeon 34036 (KR)
(72) Inventor: KOH, Seong Ho, Seongnam-si Gyeonggi-do 13523 (KR); LEE, Kyuyong, Guri-si Gyeonggi-do 11923 (KR); KIM, Sang Jae, Seoul 06360 (KR)
(74) Representative: Inspicos P/S
(86) International application number: PCT/KR2016/012613
(87) International publication number: WO 2017/078440

(57) **Abstract**

The present invention relates to a composition for neuronal loss prevention and regeneration and, more specifically, to a composition for neuronal loss prevention and regeneration, containing a telomerase-derived peptide so as to be effective on known diseases caused by neuronal loss and regeneration inability. According to the present invention, the peptide shows preventive and treatment effects on neuronal loss and regeneration inability diseases including Alzheimer's disease, and thus a novel method for treating neuronal loss and regeneration inability diseases can be provided.

## Description

### [Technical Field]

The present invention relates to a peptide effective in regeneration of neurons and prevention of neuronal loss and a composition including the same, and more particularly, to a composition for preventing and treating a cerebrovascular or neurodegenerative disease, which includes a telomerase-derived peptide and is effective in regeneration of neurons and prevention of neuronal loss, resulting in prevention and treatment of a disease caused by neuronal loss.

### [Background Art]

Neuronal cells are various types of cells generated from the differentiation of neural stem cells (NSCs). NSCs differentiate into various types such as neurons, glia, astrocytes, and oligodendrocytes, which constitute a nervous system.

There are markers indicating respective steps in differentiation of NSCs into neurons, the steps including proliferation, differentiation, migration, targeting and synaptogenesis. Doublecortin (DCX) is a protein expressed in neural progenitors, and indicates immature neurons, neuron-specific class IIIβ tubulin (TuJ1) indicates actively dividing neural progenitor cells or actively divided neurons expressed in newly generated immature post-mitotic neurons, and neuronal nuclear antigen (NeuN) is a nuclear protein expressed in post-mitotic cells and indicates mature neurons. According to the expression levels of these markers, it can be determined whether neurons are differentiated or proliferated, and normal proliferation occurs.

Neuronal damage results in various symptoms, and brain nerve cell damage may lead to cognitive impairment and macular degeneration. Among markers indicating neuronal damage, glial fibrillary acidic protein (GFAP) is a protein expressed in the central nervous system including astrocytes, and functions to form the cytoskeleton of astrocytes. Abnormal expression of this protein induces gliosis, leading to cognitive impairment. In addition, galactosylceramidase (GalC) is an enzyme that removes galactose, serves as an oligodendrocyte marker, and indicates that NSCs have differentiated into oligodendrocytes. Through these markers, it can be determined whether NSCs are damaged and oligodendrocytes are regenerated.

An abnormality in nerve function is called a neurological disease, such as stroke, brain tumors, Parkinson's disease, epilepsy, migraines, sleep disorders, or dementia.

Among neurological diseases, stroke is a cerebrovascular disease that causes brain abnormalities due to pathological changes of cerebral blood vessels, leading to an intraluminal occlusion caused by an embolism or thrombosis, or the rupture of blood vessels. Such stroke is a disease generated by blocked or burst brain blood vessels, and divided into two types such as cerebral infarction and cerebral hemorrhage. Cerebral infarction is called an ischemic cerebrovascular disease, which is caused by difficulty in blood and oxygen supply to the brain due to blockage of brain blood vessels, leading to death of brain cells. Meanwhile, cerebral hemorrhage is called a hemorrhagic cerebrovascular disease, in which the brain is damaged due to burst cerebrovascular vessels and bleeding. The cerebral infarction and cerebral hemorrhage cause the brain to lose its function, resulting in hemiplegia, speech and consciousness disorders, and in severe cases, resulting in death.

The ischemic cerebrovascular disease is a disease caused by delayed neuronal death in the hippocampus CA1 area since it is difficult to supply oxygen and glucose to brain cells due to the reduction in cerebral blood flow (Kirino, Brain Res., 239, p57-69, 1982). In addition, the ischemic brain disease is subdivided into thrombosis, embolisms, transient ischemic attacks and lacume.

The molecular biological mechanism of ischemic injury such as stroke is that when blood supply to a specific part of the parenchyma is blocked, it is impossible to supply sufficient ATP to maintain an intracellular ion channel due to the interruption of the supply of oxygen and glucose, and intracellular uptake of calcium ions is increased by inducing the continuous activation of an N-methyl-D-aspartic acid (NDMA) receptor, α-amino-3-hydroxy-5-methyl-4-isoxazole-propinoic acid (AMPA) and a metabotropic glutamate receptor (Science, 244, p1360-1362, 1989). The intracellular uptake of calcium is known to promote the death of neuronal cells by destroying cell structures such as DNA and the cell membrane by producing cytotoxic molecules including free radicals by excessive release of glutamate and aspartate.

Ischemic stroke is the No. 1 cause of death as a single disease, and requires a great deal of socioeconomic costs for treatment. Stroke is a disease with a high incidence in the elderly, but recently the incidence of stroke in the 30s to 40s accounts for approximately 1/4 of the total stroke patients, which means that the stroke is spreading to the younger ages. However, no drugs that have a critical effect on prevention of neuronal damage caused by stroke have as yet been developed. There is no distinct treatment for most patients, and thus there is only a focus on conservative treatment and treatment for secondary prevention, but serious sequelae are generated. Therefore, studies to reduce such sequelae by minimizing the death of brain cells due to stroke through the development of new therapies are being conducted. Drugs that have been developed so far for the purpose of protecting neurons from stroke are used to block certain sites of the apoptosis pathway caused by stroke. However, since neuronal apoptosis caused by stroke occurs through various pathways, such drugs are not very effective for stroke. Recently, there are progressing studies on target therapies to identify the precise pathology of ischemic stroke and minimize the death of brain nerve cells by regulating various factors involved in the pathological mechanism.

In the present invention, it was confirmed that a telomerase-derived peptide has an effect of inhibiting neuronal apoptosis caused by oxygen-glucose deprivation (OGD), which is an *in vitro* model of PEP1 ischemic stroke, known to have an anticancer or anti-inflammatory effect. In numerous clinical trials targeting various diseases, the toxicity of PEP1 has been tested, and it was identified that PEP1 is stable in terms of side effects. Until now, various drugs suggested as a therapeutic agent for ischemic stroke were very limited in terms of a treatment target or a time of occurrence, and had a side effect of hemorrhaging, but PEP1 exhibited an unexpected and remarkable neuroprotective effect.

Among neurological diseases, neurodegenerative diseases caused by the degeneration, reduction or apoptosis of brain or peripheral nerve cells, or damage or exclusion of these cells due to an environmental or genetic reason include dementia, Parkinson's disease, Alzheimer's disease, Huntington's disease, spinocerebellar degeneration, amyotrophic lateral sclerosis, polyneuropathy, spinal cord injuries, and cerebrovascular disorders. In the treatment of these neurological diseases, supplementation of neurotransmitters which are lost by an injury to neurons, or regeneration of neurons is the core of this treatment.

Dementia refers to a series of syndromes, such as a gradual decrease in cognitive function generally found in patients with brain diseases showing brain cell damage and a decrease in brain cells accompanying the damage. The decrease in brain cells is a process of aging, but diseases causing dementia lead to a faster decrease in brain cells, resulting in an abnormal brain function. According to the result of investigating the prevalence of dementia in 2012, the prevalence of dementia in the elderly over 65 years was 9.18%. The dementia prevalence keeps increasing due to rapid aging, and it is estimated that it will increase approximately two-fold every 20 years, for example, from approximately 540,000 in 2012 to approximately 1.27 million in 2030 and approximately 2.71 million in 2050. It is estimated that Alzheimer's dementia accounts for more than a half of the major causes of dementia. The second cause of dementia following Alzheimer's disease is vascular cognitive impairment including vascular dementia.

Recently, Alzheimer's disease has a trend of an increasing number of patients, and it is also a social problem that gives much suffering to patients and their families.

Alzheimer's disease is a degenerative brain disease characterized by a loss of neurons in the cerebral cortex and abnormal protein invasion, and resulting in the atrophy of the cerebral cortex (parietal lobe and temporal lobe), and is one of the most common causes of dementia. Although the etiology is still unclear, pathologically, Alzheimer's disease is characterized by infiltration of an abnormal amyloid protein and a β-amyloid plaque composed of dead neurons and inflammatory cells outside the neurons, and a concentration of a neurofibrillary tangle (NFT) consisting of a hyperphosphorylated tau protein inside the neurons.

In the early stage of neurodegeneration shown in Alzheimer's disease (AD), decreases in synaptic toxicity and neutrophils, neurotransmitter abnormalities, extracellular accumulation of β-amyloid (Aβ) (amyloid/senile plaque), intracellular NFTs, and gliosis are shown, and in the late stage thereof, significant nerve loss and associated brain atrophy are shown. In the early stage, the influence of such neurodegeneration is limited to the entorhinal cortex and the hippocampus, and cognitive and memory impairments appear. As AD progresses, up to 80% of hippocampal neurons die, and according to the progression of AD symptoms, cognitive disorders and daily life disorders are shown, resulting in the reduction of overall clinical global impression scores.

Depending on the cause of AD, AD is classified into familial AD and sporadic AD. The familial AD has been known to account for approximately 1% to 5% of cases. Most ADs do not exhibit autosomal heredity, and in the sporadic AD, environmental and genetic factors are considered to act as risk factors. In the case of familial AD, in a patient with an autosomal dominant or familial early-onset AD, the relevance between mutations in the following four genes and the onset of AD has been suggested. These four genes include: amyloid precursor protein (APP), presenilin 1 (PS1), presenilin 2 (PS2) and apolipoprotein E (ApoE). Mutations associated with APP and PS proteins may lead to an increase in the production of Aβ peptides, specifically the more amyloidogenic form, Aβ42, which is the main ingredient of senile plaques.

While the pathogenic mechanism and cause of AD have yet to be precisely revealed, a neurotransmitter hypothesis associated with a decrease or increase in the level of neurotransmitters, an amyloid hypothesis associated with β-amyloid deposition, or a tau hypothesis associated with tau hyperphosphorylation has been suggested as another pathogenic mechanism, other than genetic mutations.

Among the AD pathogenic mechanisms, the neurotransmitter hypothesis considers that two types of neurotransmitters, such as acetylcholine and glutamate, are associated with AD. The acetylcholine (Ach) activates muscles, and assists excitement, short-term memory, and learning. AD patients show a low level of Ach. The glutamate is involved in learning and memory as the most common neurotransmitter in the brain. As the brain cells of the AD patient die, an excessive amount of glutamate is released. The excessive amount of glutamate excessively stimulates healthy brain cells, and thus a phenomenon known as excitotoxicity occurs to damage or kill neurons, resulting in the exhibition of harmful effects. As AD therapies, cholinergic nervous system regulatory drugs such as donepezil (trade name: Aricept), rivastigmine (trade name: Exelon), galantamine (trade name: Razadyne) and memantine (trade name: Namenda) have a mechanism of improving a decreased signaling function, and as a glutamate neurotransmitter, the memantine having an NMDA receptor antagonist mechanism which inhibits neuronal damage is mainly used. However, these drugs have efficacy that is only sufficient to improve symptoms.

There is no drug capable of fundamentally treating AD so far. Drugs currently commercially available are effective in alleviating symptoms and delaying progression. Conventional therapeutic agents for AD, for example, donepezil, rivastigmine, galantamine and memantine have efficacy by regulating neurotransmitters. Donepezil, rivastigmine and galantamine are acetylcholineesterase inhibitor-type drugs that increase the acetylcholine concentration in the brain. These drugs are popular therapeutic agents that serve to inhibit acetylcholineesterase involved in the degradation of acetylcholine, and improve the cognitive function of an AD patient. However, as common side effects of cholinesterase inhibitors, nausea, vomiting, diarrhea, the loss of appetite, muscle cramps and sleep disorders, caused by increased acetylcholine, have been known, and these drugs merely stabilize or temporarily alleviate AD symptoms, but do not prevent the symptoms, and therefore, they are not fundamental therapeutic agents. The memantine regulates another neurotransmitter, glutamate, and serves to prevent cytotoxicity by glutamate as a non-competitive NMDA antagonist and is used to treat moderate and severe AD patients. The glutamate as an excitatory neurotransmitter activates an NMDA receptor, and when the receptor is excessively activated, due to excessive calcium uptake into brain nerve cells, the cells are destroyed. In the AD patient, as the glutamate is excessively secreted, the calcium ion channel of the NMDA receptor is always open even in the stable phase, thereby introducing an excessive amount of calcium into the cells, and the memantine serves as an antagonist with low affinity in a calcium channel located in the NMDA receptor to block intracellular uptake of calcium and prevent the destruction of brain nerve cells. However, the memantine is also known to have side effects of dizziness, headaches, etc.

The amyloid hypothesis assumes that extracellular amyloid beta (Aβ) aggregation or a reduction in Aβ removal is the main cause of a disease that causes toxic events and brings about neuron degeneration. Aβ is generated by proteolysis of a type 1 cell membrane (integral membrane) glycoprotein, amyloid precursor protein (APP). The degradation of APP into Aβ is performed by two major pathways, that is, an amyloidogenic pathway and a non-amyloidogenic pathway, and cleavage is performed with enzyme groups called (α), (β) and (γ) secretases. APP processing caused by the γ-secretase activity is involved in the final step of release of both Aβ1-40 and Aβ1-42. Each of presenilin 1 (PS1) and presenilin 2 (PS2) contributes to the formation of the secretase complex. Aβ has been known to be present in two major types such as Aβ1-40 and Aβ1-42 (both have common N-terminals or different C-terminals). In a normal environment, Aβ1-40 is formed by degradation with α-γ-secretase. Aβ1-40 has low probability of aggregation or toxicity. However, in a pathogenic environment, degradation with a β-γ-secretase occurs, producing Aβ1-42. Aβ1-42 is the most frequently found type in the brain of an AD patient. One of the pieces of evidence supporting the amyloid hypothesis is that an apolipoprotein improves the degradation of beta-amyloid, but the degradation actions of certain isoforms such as APOE4 are weak, resulting in excessive amyloid deposition in the brain. Based on the amyloid hypothesis, there was an attempt to develop an antibody targeting an amyloid beta peptide as an AD therapeutic agent. Until now, clinical phase 3 trials for bapineuzumab (Pfizer), solanezumab (Lilly) and an immunoglobulin-based drug, such as Gammagard (Baxter), which attracted attention as antibody therapeutic agents recognizing the beta amyloid peptide have all failed.

As described above, an insoluble, fibrillar aggregate of the beta amyloid is a neuropathologically representative marker for AD, but the relevance with human brain dysfunction is not clearly explained. Recently, in terms of the onset of AD, as a new hypothesis on the role of the beta amyloid, studies on the cytotoxic role of the soluble aggregate (oligomers) of the beta amyloid have been conducted. Studies on the effects of amyloid-β oligomers such as an amyloid-β dimer, an amyloid-β trimer and Aβ56 on the cognitive ability in animal models have been conducted. There is potent evidence that when the amyloid-β dimer and Aβ56 are injected into healthy young rats, the brain function is damaged.

The tau hypothesis assumes that tau hyperphosphorylation is the main cause of AD. In the brain of an AD patient, hyperphosphorylated tau is the main component of NFTs. An NFT is a fibrous aggregate containing tau in AD and other tau-associated diseases. In normal environments, tau is soluble, and is generally found in axons that maintain the assembly and stability of microtubular and vesicular transport. A microtubule-associated tau protein has been reported to play a major role in normal neural activation in the mammalian brain. Meanwhile, in AD and tau-associated pathological environments, due to an imbalance between the activities of a tau kinase and a phosphatase, tau hyperphosphorylation occurs, and the morphology of tau is changed. According to such a change, the tau protein becomes insoluble, has a reduced affinity to a microtubule, and is detached from the microtubule, resulting in the formation of a double-helix filament structure by self-aggregation. These filaments aggregate to NFTs, and interrupt and inhibit the axon transport. Approximately 30 or more types of mutations found at chromosome 17 at which a gene encoding tau, microtubule-associated protein tau (MAPT), is located are associated with familial AD (FAD) and Parkinson's disease. In addition, the mutations confirmed in MAPT reduce the attachment of the tau protein to the microtubule, and increase a tendency of aggregation to an abnormal filament. This corresponds to the tau pathological symptom in AD. Such result supports the hypothesis in that a dysfunctional tau protein is the main cause of a neurodegenerative disease.

The confirmation of familial AD (FAD) mutations has allowed the formation of an AD animal model. Currently used animal models have been developed using mutated APP, PS1 and MAPT genes.

As animal models used in effect and efficacy tests for developing therapeutic agents for neurodegenerative diseases including AD, there are mice (3XTg-AD mouse models) in which specific three genes (APP, Tau, PS1) are mutated to deposit amyloid beta and induce NFTs. The mice with the three mutated genes show the progression of neuronal damage-associated diseases including AD symptoms over time. It has been known that, after a developed drug is injected into the neuronal damage-induced animals, neuronal destruction inhibitory and regenerative effects were observed, and compared with those of a non-injected control group, thereby measuring the effects and efficacies of therapeutic agent candidates.

The peptide according to the present invention (hereinafter, referred to as PEP1) is a peptide consisting of 16 major amino acids present in a catalytic region of telomerase, and has been known to have anti-inflammatory and antioxidative effects. As it is revealed that the peptide according to the present invention is effective in prevention and restoration of neuronal loss that is the fundamental cause of a neurodegenerative disease through animal tests, it is expected that PEP1 will show effects of preventing and treating a neurological disease beyond the limits of a conventional therapeutic agent in the prevention and treatment of a neurodegenerative disease, that is, simple alleviation of symptoms.

The present invention provides a composition for preventing and restoring neuronal loss, which includes a peptide derived from a reverse transcriptase of telomerase. Specifically, the present invention provides a composition for preventing and restoring neuronal loss, which includes a peptide derived from reverse transcriptase of human telomerase (hTERT), which consists of amino acids. More specifically, the present invention provides a hTERT-derived peptide consisting of 16 amino acids, PEP1, as a composition for preventing and restoring neuronal loss.

### [Prior Art Document]

WO 2010128807 A2

### [Disclosure]

### [Technical Problem]

Against this background, the inventors made an effort to develop a composition for preventing and restoring neuronal loss, which is very effective in prevention and restoration of neuronal loss without side effects, and therefore, completed the present invention.

The present invention is directed to providing a composition for preventing and restoring neuronal loss, which is a fundamental therapeutic agent effective for a neurodegenerative disease caused by neuronal loss without side effects.

### [Technical Solution]

According to an aspect of the present invention, a composition for preventing and restoring neuronal loss, which includes a pharmaceutically effective amount of one or more selected from the group consisting of a peptide comprising an amino acid sequence of SEQ ID NO: 1, a peptide having at least 80% sequence homology with the amino acid sequence, and a fragment thereof, is provided.

According to another aspect of the present invention, one or more selected from the group consisting of a peptide comprising an amino acid sequence of SEQ ID NO: 1, a peptide having at least 80% sequence homology with the amino acid sequence, and a fragment thereof is provided to be used in prevention of neuronal loss and regeneration of neurons.

According to still another aspect of the present invention, a use of one or more selected from the group consisting of a peptide comprising an amino acid sequence of SEQ ID NO: 1, a peptide having at least 80% sequence homology with the amino acid sequence, and a fragment thereof is provided to prepare a composition for preventing and restoring neuronal loss.

Regarding the composition according to the present invention, the fragment may be a fragment consisting of three or more amino acids.

Regarding the composition according to the present invention, the composition is for preventing, treating, alleviating and improving a disease, or for preventing, treating, alleviating and improving sequelae of the disease.

Regarding the composition according to the present invention, the disease caused by neuronal loss and regeneration inability may be a cerebrovascular disease or a neurodegenerative disease.

Regarding the composition according to the present invention, the cerebrovascular disease may be an ischemic cerebrovascular disease or a hemorrhagic cerebrovascular disease.

Regarding the composition according to the present invention, the neurodegenerative disease may be any one of cerebropathia, spinal cord injuries, peripheral nerve damage, a peripheral nerve disease, amyotrophic lateral sclerosis, dementia, Huntington's disease, Parkinson's disease, an AD-like disease, spinocerebellar degeneration, and polyneuropathy.

Regarding the composition according to the present invention, the composition maybe co-administered with one or more therapeutic agents for a neurological disease.

Regarding the composition according to the present invention, the AD-like disease may exhibit any one or more of dementia, cognitive disorders, and memory loss.

Regarding the composition according to the present invention, the AD-like disease may be an early, progressive or severe AD-like disease.

Regarding the composition according to the present invention, the peptide of SEQ ID NO: 1 may be administered as a pharmaceutically active ingredient at a dose of 0.0001 to 100 mg/kg/day.

Regarding the composition according to the present invention, the peptide of SEQ ID NO: 1 may be administered as a pharmaceutically active ingredient at a dose of 0.01 to 200 mg.

Regarding the composition according to the present invention, the peptide of SEQ ID NO: 1 may be administered as a pharmaceutically active ingredient once every 1 to 4 weeks for a total of four or more times.

Regarding the composition according to the present invention, the composition including the peptide of SEQ ID NO: 1 as a pharmaceutically active ingredient may be administered orally, intrarectally, percutaneously, intravenously, intramuscularly, intraperitoneally, intramedullarily, intrathecally, intracutaneously, or subcutaneously.

According to yet another aspect of the present invention, a method for preventing and restoring neuronal loss, which includes administering a pharmaceutically effective amount of the composition according to the present invention to a subject that requires the composition, is provided.

According to yet another aspect of the present invention, a kit for preventing and restoring neuronal loss, which includes a protocol that indicates administering the composition for preventing and restoring neuronal loss according to the present invention so as to contain 0.01 to 200 mg of the peptide of SEQ ID NO: 1 as a pharmaceutically active ingredient to a subject requiring the composition once every 1^{st} to 4^{th} week, is provided.

### [Advantageous Effects]

Since a peptide having a sequence of the sequence number of the present invention, a peptide having a sequence with 80% homology with the sequence or a fragment thereof is effective in prevention of neuronal loss and regeneration of neurons, the present invention provides a method for preventing and treating a disease caused by neuronal loss or regeneration inability.

### [Description of Drawings]

FIG. 1 is a graph showing the results of measuring cell viability using a cell count kit-8 (CCK-8)) and cell activity using BrdU staining when NSCs are treated with Aβ25-35 (β-Amyloid 25-35) at each concentration (0, 2.5, 5, 10, 20, or 40 µM), relative to the control group (0 µM).
FIG. 2 is a graph showing the results of measuring cell viability using CCK-8 and cell activity using BrdU staining when NSCs are treated with PEP1 at each concentration (0, 1, 10, 50, 100, or 200 µM), relative to the control group (0 µM).
FIG. 3 is an absorbance graph showing the result of measuring cell viability using CCK-8, MTT, and LDH when NSCs are treated with Aβ25-35 at 0 µM (control) and 20 µM and PEP1 at each concentration (0, 1, 10, 50, or 100 µM), relative to the control group.
FIG. 4 is an absorbance graph showing the result of measuring cell proliferation when NSCs are treated with Aβ25-35 at 0 µM (control) and 20 µM and PEP1 at each concentration (0, 1, 10, 50, or 100 µM).
FIG. 5 is an image showing apoptosis confirmed by DAPI and TUNEL staining when NSCs are treated with Aβ25-35 at 0 µM (control) and 20 µM and PEP1 at each concentration (0, 1, 10, 50, or 100 µM).
FIG. 6 is a graph showing cell colony counts measured by a colony-forming unit (CFU) counting method when NSCs are treated with Aβ25-35 at 0 µM (control) and 20 µM and PEP1 at each concentration (0, 1, 10, or 50 µM).
FIG. 7 is an image showing apoptosis before and after treatment, evaluated by TUNEL staining, when NSCs are treated with Aβ25-35 at 0 µM (control) and 20 µM and PEP1 at each concentration (0, 1, 10, 50, or 100 µM).
FIG. 8 is an absorbance graph showing percentages of apoptotic cells before and after treatment, evaluated by TUNEL staining, when NSCs are treated with Aβ25-35 at 0 µM (control) and 20 µM and PEP1 at each concentration (0, 1, 10, 50, or 100 µM).
FIG. 9 is a graph showing the result of a cell migration assay using a QCM-24 well cell migration kit to observe cell migration ability when NSCs are treated with Aβ25-35 at 0 µM (control) and 20 µM and PEP1 at each concentration (0, 1, 10, or 50 µM).
FIGS. 10 and 11 are images of the results of mitochondrial staining and cell membrane staining after PEP1 is labeled with FITC and treated for 0 to 24 hours to observe a process by which PEP1 penetrates into NSCs after treatment of 10 µM of PEP1.
FIGS. 12 and 13 are images of the results of nuclear staining after PEP1 is labeled with FITC and treated for 0 to 24 hours to observe a process by which PEP1 penetrates into β-Amyloid-treated NSCs after NSCs that have been treated with 20 µM of Aβ25-35 are treated with 1 µM of PEP1.
FIGS. 14 to 16 are graphs showing the results of measuring DCF fluorescence values after DCFH-DA staining to detect production of reactive oxygen species (ROS) when NSCs are treated with Aβ25-35 at 0 µM (control) and 20 µM and PEP1 at each concentration (0, 1, 10, or 50 µM).
FIG. 17 is a graph showing the result of evaluating a degree of oxidative DNA damage of a mitochondria using an 8-OHdG ELISA kit when NSCs are treated with Aβ25-35 at 0 µM (control) and 20 µM and PEP1 at each concentration (0, 1, 10, or 50 µM).
FIG. 18 is a graph showing the result of observing a mitochondria membrane potential using an ELISA reader when NSCs are treated with Aβ25-35 at 0 µM (control) and 20 µM and PEP1 at each concentration (0, 1, 10, or 50 µM).
FIGS. 19 and 20 are the result of observing mitochondria cell membrane potential using a JC-1 kit when NSCs are treated with Aβ25-35 at 0 µM (control) and 20 µM and PEP1 at each concentration (0, 1, 10, or 50 µM), in which dead cells or unhealthy cells are observed at a FITC wavelength (FIG. 19) and healthy cells are observed at a rhodamine wavelength and a Texas red wavelength (FIG. 20).
FIG. 21 is a graph showing ATP concentrations (nmol/µL) when NSCs are treated with Aβ25-35 at 0 µM (control) and 20 µM and PEP1 at each concentration (0, 1, 10, or 50 µM).
FIG. 22 is an image showing the result of an antibody microarray performed to evaluate the effect of PEP1 on expression levels of intracellular proteins in a proteomic aspect when NSCs are treated with Aβ25-35 at 0 µM (control) and 20 µM and PEP1 at each concentration (0, 1, 10, or 50 µM).
FIG. 23 is table that shows expressed and decreased intracellular proteins under each condition when NSCs are treated with Aβ25-35 at 0 µM (control) and 20 µM and PEP1 at each concentration (0, 1, 10, or 50 µM).
FIG. 24 is a set of images of the result of a microarray to evaluate expression levels of intracellular proteins when NSCs are treated with Aβ25-35 at 0 µM (control) and 20 µM, and PEP1 at each concentration (0 or 10 µM).
FIG. 25 is a table showing expressed and decreased intracellular proteins when NSCs are treated with Aβ25-35 at 0 µM (control) and 20 µM, and PEP1 at each concentration (0 or 10 µM).
FIGS. 26 and 27 are a graph (FIG. 26) showing expression of Ki67, PI3K, Ser473, Ser9 and HMGB-1, evaluated using immunostaining when NSCs are treated with Aβ25-35 at 0 µM (control) and 20 µM and PEP1 at each concentration (0, 1, 10, or 50 µM), and a graph (FIG. 27) of the digitized results.
FIGS. 28 and 29 are images (FIG. 28) showing expression of Bax, cytochrome c and caspase-3, evaluated using immunostaining when NSCs are treated with Aβ25-35 at 0 µM (control) and 20 µM and PEP1 at each concentration (0, 1, 10, or 50 µM), and a graph (FIG. 29) of the digitized results.
FIG. 30 is an image showing the expression of HMGB-1 over time when NSCs are treated with Aβ25-35 at 0 µM (control) and 20 µM, and PEP1 at each concentration (0, 1, or 10 µM).
FIGS. 31 and 32 are an image (FIG. 31) showing the result of measuring expression of PI3K, Ser473, Akt, Ser9 and Bcl-2 using immunostaining when NSCs are treated with Aβ25-35 at 0 µM (control) and 20 µM, and PEP1 at each concentration (0, 1, or 10 µM), and a graph of the digitized results (FIG. 32).
FIGS. 33 and 34 are an image (FIG. 33) showing the result of measuring expression of PARP, Bax, cytochrome-c, caspase-9 and caspase-3 using immunostaining when NSCs are treated with Aβ25-35 at 0 µM (control) and 20 µM, and PEP1 at each concentration (0, 1, or 10 µM), and a graph of the digitized results (FIG. 34).
FIG. 35 is a graph showing the result of observing cell viability when NSCs are treated with Aβ25-35 at 0 µM (control) and 20 µM, and co-treated with PEP1 at each concentration (0, 1 or 10 µM) and then with LY294002 known as a PIK3 inhibitor.
FIGS. 36 to 48 are images and graphs showing expression of Nestin, Ki67, GFAP, NeuN, DCX and Tuj1, which are markers for differentiation and division steps of NSCs, using DAPI staining and immunostaining, in the presence or absence of basic FGF (bFGF) when NSCs are treated with Aβ25-35 at 0 µM (control) and 20 µM and co-treated with PEP1 at each concentration (0 or 1 µM).
FIG. 49 is a set of images showing a shuttle vector and TERT, tagged with GFP, after 1 µg of hTERT is overexpressed in human NSCs.
FIGS. 50 to 54 show a microarray image that compares 1 µg hTERT overexpression with 1 µM PEP1 treatment, after human NSCs are treated with 20 µM of Aβ25-35 (FIG. 50), an electrophoresis image showing TERT expression after a shuttle vector and hTERT are tagged with GFP (FIG. 51), lists showing proteins increased or decreased by overexpressed hTERT and PEP1 (FIG. 52), an image showing TERT expression after human NSCs are treated with Aβ25-35 at 0 or 20 µM, and co-treated with PEP1 at each concentration (0, 1, 10, or 50 µM) (FIG. 53), and a graph of the digitized results (FIG. 54).
FIG. 55 is an image of a western blotting result showing NeuN expression in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 56 is an image of a western blotting result showing DCX expression in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results (FIG. 56).
FIG. 57 is an image showing DCX and NeuN expression by immunostaining nerve tissue in the subventricular zone (SVZ) of animal models of control groups (saline and 1 mg/kg of scramble peptide) and groups treated with different concentrations of PEP1 (0.01, 0.1 and 1 mg/kg), and a conventional drug, donepezil (1 mg/kg).
FIG. 58 is an image of a western blotting result showing Tuj1 expression in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 59 is an image showing Tuj1 expression by immunostaining nerve tissue in the subventricular zone (SVZ) of animal models of control groups (saline and 1 mg/kg of scramble peptide) and groups treated with different concentrations of PEP 1 (0.01, 0.1 and 1 mg/kg), and a conventional drug, donepezil (1 mg/kg) with nerve tissue.
FIG. 60 is an image of a western blotting result showing GFAP expression in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 61 is an image of a western blotting result showing GalC expression in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 62 is an image showing DCX and NeuN expression by immunostaining the SVZ of animal models of control groups (saline and 1 mg/kg of scramble peptide) and groups treated with different concentrations of PEP1 (0.01, 0.1 and 1 mg/kg), and a conventional drug, donepezil (1 mg/kg) with nerve tissue.
FIG. 63 is an image of a western blotting result showing expression of monomer β-Amyloids in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 64 is an image of a western blotting result showing expression of oligomer β-Amyloids in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 65 is an image of a western blotting result showing expression of tau, which is a marker indicating NFT generation, in the hippocampus and the whole brain of animal models of control groups (saline and 1 mg/kg of scramble peptide) and groups treated with different concentrations of PEP1 (0.01, 0.1 and 1 mg/kg), and a conventional drug, donepezil (1 mg/kg), and a graph of the digitized results.
FIG. 66 is an image showing expression of NFT (measured with Tau) and β-Amyloids in neurons by immunostaining nerve tissue of animal models of control groups (saline and 1 mg/kg of scramble peptide) and groups treated with different concentrations of PEP1 (0.01, 0.1 and 1 mg/kg), and a conventional drug, donepezil (1 mg/kg).
FIG. 67 is a graph showing the results of passive avoidance tests for animal models of control groups (saline and 1 mg/kg of scramble peptide) and groups treated with different concentrations of PEP1 (0.01, 0.1 and 1 mg/kg), and a conventional drug, donepezil (1 mg/kg), evaluated as good (hatched) and bad (not hatched).
FIG. 68 is a graph showing a response time (units of sec) in a passive avoidance test for animal models of control groups (saline and 1 mg/kg of scramble peptide) and groups treated with different concentrations of PEP1 (0.01, 0.1 and 1 mg/kg), and a conventional drug, donepezil (1 mg/kg).
FIG. 69 is a graph showing the result of a Y-maze test for animal models of control groups (saline and 1 mg/kg of scramble peptide) and groups treated with different concentrations of PEP 1 (0.01, 0.1 and 1 mg/kg).
FIG. 70 is a graph showing the result of a passive avoidance test for severe AD animal models to evaluate a ratio of the number of subjects staying in a bright room.
FIG. 71 is a graph showing the result of a Y-maze test for severe AD animal models to evaluate spontaneous alteration.

### [Modes of the Invention]

The present invention may have various modifications and embodiments, and thus the present invention will be described in further detail below. However, the present invention is not limited to specific embodiments, and it should be understood that the present invention includes all modifications, equivalents and alternatives included in the technical idea and scope of the present invention. To explain the present invention, if it is determined that a detailed description of the related art may obscure the gist of the present invention, the detailed description thereof will be omitted.

In neurological diseases, neuronal degeneration, depletion or apoptosis causes various pathological symptoms. Diseases caused by neuronal loss and regeneration inability include cerebrovascular diseases such as ischemic stroke and neurodegenerative diseases such as dementia and AD. Therapeutic agents for neurodegenerative diseases, which have been developed until now, are only drugs for improving symptoms, but may not change the progression of a disease. For this reason, there is a demand for developing a neuroprotective drug or nerve regeneration therapy as a fundamental therapy. In addition, drugs that have been developed to protect neurons from ischemic stroke are designed to block only a specific part of an apoptotic pathway induced by stroke, but the apoptosis of neurons caused by stroke is accomplished by various pathways. Therefore, these drugs do not offer a fundamental solution. For this reason, there is a demand for a target therapy that minimizes the death of brain cells by simultaneously adjusting various factors involved in ischemic stroke.

A telomere, which is a repetitive genetic material located at each terminus of a chromosome, is known to prevent damage to a corresponding chromosome or coupling to a different chromosome. The telomere is gradually shortened with cell divisions, becoming very short after a certain number of cell divisions, and the cell eventually stops being divided and dies. On the other hand, the elongation of telomeres is known to extend the life span of a cell. As an example, it has been known that, in cancer cells, an enzyme called telomerase is secreted to prevent the shortening of telomeres, resulting in steady proliferation of the cancer cells, without death. While PEP1, a telomerase-derived peptide, has been developed as an anticancer agent for various types of cancer including pancreatic cancer, it has been revealed that PEP1 is involved in antiinflammation, antioxidation, cell permeability and major intracellular signaling as well as having an anticancer effect. In view of the intracellular functions of PEP1, the possibility of using new indications in addition to its use as an existing anticancer agent has been investigated. The inventors confirmed that a telomerase-derived peptide has a mechanism of inhibiting an inflammatory action causing a neuronal disease and is effective in prevention of neuronal loss and regeneration of neurons, and thus the present invention was completed. More specifically, in the present invention, through an AD animal model test, it was confirmed that PEP1 reduces aggregation of β amyloids, which is presumed to be the cause of AD, and decreases NFTs formed by the hyperphosphorylation of a tau protein. Moreover, in the present invention, it was confirmed that PEP1 has a positive effect on differentiation and proliferation of neurons through the expression levels of various neuron markers.

In an aspect of the present invention, the peptide of SEQ ID NO: 1 (PEP1), the fragment of the peptide of SEQ ID NO: 1 or the peptide having at least 80% sequence homology with the peptide sequence includes a peptide derived from a telomerase, and specifically, *Homo sapiens* telomerase.

The peptide disclosed herein may include peptides having at least 80%, 85%, 90%, 95%, 96%, 97%, 98% or 99% sequence homology. In addition, the peptide described herein may include a peptide having a difference in 1 or more amino acids, 2 or more amino acids, 3 or more amino acids, 4 or more amino acids, 5 or more amino acids, 6 or more amino acids, or 7 or more amino acids from the peptide of SEQ ID NO: 1 or fragments thereof.

In an aspect of the present invention, the change in amino acid is a property of changing the physicochemical characteristics of a peptide. For example, the change in amino acid may be made to improve thermal stability, alter substrate specificity, and change the optimal pH of a peptide.

The term "amino acid" used herein not only includes the 22 standard amino acids that are naturally incorporated into a peptide, but also includes the D-isomers and modified amino acids. Therefore, in one aspect of the present invention, the peptide may be a peptide including a D-amino acid. On the other hand, in another aspect of the present invention, the peptide may include a non-standard amino acid, which is subjected to post-translational modification. Examples of the post-translational modification include phosphorylation, glycosylation, acylation (e.g., acetylation, myristoylation, and palmitoylation), alkylation, carboxylation, hydroxylation, glycation, biotinylation, ubiquitination, a change in chemical properties (e.g. β-removing deimidation and deamidation), and a structural change (e.g. formation of a disulfide bridge). The post-translational modification also includes changes of amino acids occurring due to chemical reactions during coupling with crosslinkers for formation of a peptide conjugate, for example, a change in an amino acid such as a change in an amino group, a carboxyl group, or a side chain.

The peptide disclosed herein may be a wild-type peptide identified and isolated from a natural source. Meanwhile, the peptide disclosed in the specification may be an artificial variant comprising an amino acid sequence in which one or more amino acids are substituted, deleted, and/or inserted compared with the fragments of the peptide of SEQ ID NO: 1. The changing of amino acids in the wild-type polypeptide, as well as the artificial variant, includes substitutions of conservative amino acids, which do not have a significant influence on folding and/or activity of a protein. The conservative substitution may be carried out within the range of the group consisting of basic amino acids (arginine, lysine and histidine), acidic amino acids (glutamic acid and aspartic acid), polar amino acids (glutamine and asparagine), hydrophobic amino acids (leucine, isoleucine, valine and methionine), aromatic amino acids (phenylalanine, tryptophan and tyrosine), and small amino acids (glycine, alanine, serine and threonine). Generally, amino acid substitutions that do not change specific activities are known in the art. The most frequently-occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly, and vice versa. Other examples of the conservative substitution are shown in the following table.

**[Table 1]**

| Original amino acid | Exemplary residue substitution | Preferred residue substitution |
|---|---|---|
| Ala (A) | val; leu; ile | Val |
| Arg (R) | lys; gln; asn | Lys |
| Asn (N) | gin; his; asp, lys; arg | Gln |
| Asp (D) | glu; asn | Glu |
| Cys (C) | ser; ala | Ser |
| Gln (Q) | asn; glu | Asn |
| Glu (E) | asp; gln | Asp |
| Gly (G) | Ala | Ala |
| His (H) | asn; gln; lys; arg | Arg |
| Ile (I) | leu; val; met; ala; phe; norleucine | Leu |
| Leu (L) | norleucine; ile ; val; met; ala; phe | Ile |
| Lys (K) | arg; gin; asn | Arg |
| Met (M) | leu; phe; ile | Leu |
| Phe (F) | leu; val; ile; ala; tyr | Tyr |
| Pro (P) | Ala | Ala |
| Ser (S) | thr | Thr |
| Thr (T) | Ser | Ser |
| Trp (W) | tyr; phe | Tyr |
| Tyr (Y) | trp; phe ; thr; ser | Phe |
| Val (V) | ile; leu; met; phe; ala; norleucine | Leu |

In terms of biological properties of the peptide, a substantial modification is performed by selecting a substituent which has a considerably different effect in (a) maintaining the backbone structure, for example, a sheet- or helix-like three-dimensional structure, of the polypeptide in a substituted region, (b) maintaining charge or hydrophobicity of the molecule at a target site, or (c) maintaining the bulk of a side chain. Natural residues are classified into the following groups, based on general properties of the side chain:
(1) Hydrophobic: norleucine, met, ala, val, leu, ile;
(2) Neutral hydrophilic: cys, ser, thr;
(3) Acidic: asp, glu;
(4) Basic: asn, gln, his, lys, arg;
(5) Residues affecting chain conformation: gly, pro; and
(6) Aromatic: trp, tyr, phe.

Non-conservative substitutions may be performed by exchanging a member of one of the groups for that of another group. Any cysteine residue, which is not associated with maintaining the proper three-dimensional structure of the peptide, may typically be substituted with serine, thus increasing the oxidative stability of the molecule and preventing abnormal crosslinking, and, conversely, enhanced stability can be achieved by adding cysteine bond(s) to the peptide.

A different type of amino acid variant of the peptide is made by changing a glycosylation pattern of an antibody. The term "change" used herein refers to deletion of one or more carbohydrate residues that are found on the peptide and(or) addition of one or more glycosylation sites which are not present in the peptide.

Glycosylation in peptides is typically N- or O-linked glycosylation. The term "N-linked glycosylation" used herein refers to attachment of carbohydrate residues to side chains of asparagine residues. As tripeptide sequences, asparagine-X-serine and asparagine-X-threonine (where the X is any amino acid, excluding proline) are recognition sequences for enzymatically attaching a carbohydrate residue to a side chain of an asparagine. Therefore, when one of these tripeptide sequences is present in a polypeptide, a potential glycosylation site is created. The "O-linked glycosylation" used herein refers to the attachment of one of the saccharides, for example, N-acetylgalactosamine, galactose, or xylose, to hydroxyamino acids and, most typically, to serine or threonine, but 5-hydroxyproline or 5-hydroxylysine may also be used.

Addition of a glycosylation site to the peptide is conveniently performed by changing the amino acid sequence to contain the tripeptide sequence described above (for an N-linked glycosylation site). Such a change may be made by addition of one or more serine or threonine residues to the initial antibody sequence or by substitution with one of these residues (for an O-linked glycosylation site).

Also, the peptide comprising the sequence of SEQ ID NO: 1 according to an aspect of the present invention, a fragment of the sequence of SEQ ID NO: 1 or a peptide having at least 80% sequence homology with the peptide has low cytotoxicity and high *in vivo* stability. In the present invention, SEQ ID NO: 1 represents a telomerase-derived peptide, which is a peptide consisting of 16 amino acids as follows.

The peptide set forth in SEQ ID NO: 1 is shown in Table 2 below. The "name" in Table 2 below is given to distinguish one peptide from another. In one aspect of the present invention, the peptide set forth in SEQ ID NO: 1 represents the whole peptide of *Homo sapiens* telomerase. In another aspect of the present invention, the peptide of SEQ ID NO: 1, a fragment thereof or a peptide having at least 80% sequence homology with the peptide sequence includes a "synthetic peptide" synthesized from a peptide present at a corresponding location of the peptides included in the telomerase. SEQ ID NO: 2 denotes the full-length amino acid sequence of the telomerase.

**[Table 2]**

| SEQ. ID. NO: | Name | Location on telomerase | Sequence | Length |
|---|---|---|---|---|
| 1 | PEP1 | [611-626] | EARPALLTSRLRFIPK | 16 aa |
| 2 | | [1-1132] | | 1132 aa |
| | | | | |

In an aspect of the present invention, a pharmaceutical composition, which includes a peptide effective in prevention of neuronal loss and regeneration of neurons, for example, a peptide comprising an amino acid sequence of SEQ ID NO: 1, a peptide having at least 80% sequence homology with the amino acid sequence or a fragment thereof, as an active ingredient, is provided.

The pharmaceutical composition effective in prevention of neuronal loss and regeneration of neurons according to an aspect of the present invention may contain a peptide comprising an amino acid sequence of SEQ ID NO: 1, a peptide having at least 80% sequence homology with the amino acid sequence or a fragment thereof at a concentration of 0.01 to 100 mg/mL, and preferably 0.1 to 10 mg/mL, but if there is a difference in effect according to a dose per treatment target, the dose may be suitably adjusted. When the peptide of the present invention is contained at a content within the above-mentioned range or lower, it may be suitable for exhibiting a desired effect of the present invention, and both stability and safety of the composition may be satisfied. In addition, the above-mentioned content may be suitable in terms of cost effectiveness.

The composition according to an aspect of the present invention may be applied to all animals including a human, a dog, a chicken, a pig, a cow, a sheep, a guinea pig, and a monkey.

In an aspect of the present invention, a pharmaceutical composition which includes a peptide effective in prevention of neuronal loss and regeneration of neurons, for example, a peptide comprising an amino acid sequence of SEQ ID NO: 1, a peptide having at least 80% sequence homology with the amino acid sequence or a fragment thereof, is provided. The pharmaceutical composition according to an aspect of the present invention may be administered orally, intrarectally, percutaneously, intravenously, intramuscularly, intraperitoneally, intramedullarily, intrathecally, intracutaneously or subcutaneously, preferably, intraperitoneally, intracutaneously, intravascularly or subcutaneously, and more preferably, subcutaneously.

Dosage forms for oral administration may include, but are not limited to, tablets, pills, soft or hard capsules, granules, powders, solutions, and emulsions. Dosage forms for parenteral administration may include, but are not limited to, injections, drips, lotions, ointments, gels, creams, suspensions, emulsions, suppositories, patches or sprays.

The pharmaceutical composition according to an aspect of the present invention may comprise, if necessary, additives such as diluents, excipients, lubricants, binders, disintegrating agents, buffers, dispersants, surfactants, coloring agents, fragrances or sweetening agents. The pharmaceutical composition according to one aspect of the present invention may be prepared by a conventional method in the art.

The active ingredient of the pharmaceutical composition according to an aspect of the present invention may vary according to the patient's age, sex, weight, pathological condition and severity, administration route, or a prescriber's judgment. A dosage of the composition is determined based on such parameters by one of ordinary skill in the art, a daily dose may be, for example, 0.0001 to 100 mg/kg/day, preferably 0.001 to 10 mg/kg/day, and more preferably 0.001 to 1 mg/kg/day, and if there is a difference in effect according to a dose, the dose may be suitably adjusted. The pharmaceutical composition according to an exemplary embodiment of the present invention may be administered once to three times a day, but the present invention is not limited thereto.

The administration dose and interval of the active ingredient of the pharmaceutical composition according to an aspect of the present invention are determined by those of ordinary skill in the art. For example, the administration dose may be 0.01 to 200 mg, preferably 0.1 to 5 mg, more preferably 0.4 to 1.2 mg, further more preferably 0.4 mg, 0.56 mg, or 1.12 mg, and the administration interval may be once every 1 to 4 weeks for a total of four or more times, and preferably once every 1 to 2 weeks for a total of 6 to 26 times, but the present invention is not limited thereto.

In an aspect of the present invention, a composition for preventing and restoring neuronal loss, which includes a peptide comprising an amino acid sequence of SEQ ID NO: 1, peptide having at least 80% sequence homology with the amino acid sequence, or a fragment thereof as an active ingredient, is provided.

A dosage form of the composition according to an aspect of the present invention is not particularly limited, but may be, for example, tablets, pills, soft or hard capsules, granules, a powder, a solution, or an emulsion. Each dosage form may be prepared by properly selecting and mixing ingredients conventionally used in the corresponding field, in addition to the active ingredient, by one of ordinary skill in the art without difficulty according to a dosage form or the purpose of use, and may have a synergistic effect when being simultaneously used with other raw materials.

The terms used in the specification are intended to be used to describe specific embodiments rather than to limit the present invention. Terms without numbers in front are not intended to limit the quantity but to indicate the presence of at least one article mentioned herein. Terms "comprising", "having", "including", and "containing" should be interpreted openly (i.e. "including but not limited to").

The mentioning of a numerical range replaces mentioning of individual numbers within the range, and unless mentioned otherwise, each number is applied to the specification as if individually mentioned in the specification. The end values of all of the ranges are included in the range and can be independently combined.

All methods mentioned in the specification may be performed in a suitable order unless noted otherwise or explicitly contradicted by the context. The use of any one embodiment and all embodiments, or exemplary language (e.g., "such as" or "like to"), unless included in the claims, is used to more clearly describe the present invention rather than to limit the scope of the present invention. Any language herein outside of the claims should not be interpreted as essential to the practice of the present invention. Unless defined otherwise, technical and scientific terms used herein each has a meaning ordinarily understood by those of ordinary skill in the art to which the present invention belongs.

The exemplary embodiments of the present invention include the best modes known to the inventors for performing the present invention. Variations in the exemplary embodiments can become clear to those skilled in the art when reading the above descriptions. The inventors expect that those skilled in the art will suitably use such variations, and implement the present invention in a manner different from that described in the specification. Thus, the present invention, as allowed by the patent law, includes equivalents and all modifications of the gist of the present invention mentioned in the accompanying claims. Moreover, all possible variations with any combination of the above-mentioned components are included in the present invention, unless explicitly stated otherwise or contradicted by the context. Although the present invention is described and shown by exemplary embodiments, those skilled in the art will understand well that there can be various changes in the form and details without departing from the spirit of the invention and range defined by the claims below.

Hereinafter, the configuration and effects of the present invention will be described in further detail with reference to examples and experimental examples. However, the following examples and experimental examples are merely provided to illustrate the present invention to help understanding the present invention, and the scope of the present invention is not limited thereto.

### Example 1: Synthesis of peptide

### 1. Synthesis of peptide

A peptide of SEQ ID NO: 1 (hereinafter, referred to as "PEP1") was prepared according to a conventionally known method of solid phase peptide synthesis. Specifically, peptides were synthesized by coupling each amino acid to another from the C-terminus through Fmoc solid phase peptide synthesis (SPPS) using ASP48S (Peptron, Inc., Daejeon, Korea). Peptides in which the first amino acid of the C-terminus was attached to a resin were used:

NH₂-Lys(Boc)-2-chloro-trityl resin

NH₂-Ala-2-chloro-trityl resin

NH₂-Arg(Pbf)-2-chloro-trityl resin

In all amino acid ingredients used in the synthesis of the peptides, the N-terminus was protected with Fmoc, and the residues were protected with Trt, Boc, t-butylester (t-Bu), and 2,2,4,6,7-pentamethyl dihydro-benzofuran-5-sulfonyl (Pbf), which can be removed in an acid. Examples of the amino acid ingredients are as follows:
Fmoc-Ala-OH, Fmoc-Arg(Pbf)-OH, Fmoc-Glu(OtBu)-OH, Fmoc-Pro-OH, Fmoc-Leu-OH, Fmoc-Ile-OH, Fmoc-Phe-OH, Fmoc-Ser(tBu)-OH, Fmoc-Thr(tBu)-OH, Fmoc-Lys(Boc)-OH, Fmoc-Gln(Trt)-OH, Fmoc-Trp(Boc)-OH, Fmoc-Met-OH, Fmoc-Asn(Trt)-OH, Fmoc-Tyr(tBu)-OH, Fmoc-Ahx-OH, Trt-Mercaptoacetic acid.

As a coupling reagent, 2-(1H-benzotriazol-1-yl)-1,1,3,3-tetramethyluronium hexafluorophosphate (HBTU)/ N-hydroxybenzotriazole (HOBt)/ 4-methylmorpholine (NMM) was used. Fmoc deprotection was carried out using 20% piperidine in DMF. To isolate the synthesized peptide from the resin and to remove the protecting group of the residue, a cleavage cocktail [trifluoroacetic acid (TFA) /triisopropylsilane (TIS) /ethanedithiol (EDT) /H₂O=92.5/2.5/2.5/2.5] was used.

Each peptide was synthesized by a repeated process of reacting each corresponding amino acid using a state in which the starting amino acid was protected by the amino acid protecting group while being bound to a solid phase scaffold, washing the resulting product with a solvent, and performing deprotection. After being cleaved from the resin, the synthesized peptide was purified by HPLC, synthesis was confirmed by mass spectrometry (MS), and lyophilization was performed.

Purities of all peptides used in this example were 95% or higher, as determined using high-performance liquid chromatography.

A specific process of preparing the peptide PEP1 was as follows.
1) Coupling
   The amino acid (8 equiv.) protected with the NH₂-Lys(Boc)-2-chloro-trityl resin was mixed with the coupling reagent HBTU (8 equiv.)/HOBt (8 equiv.)/NMM (16 equiv.) which were dissolved in DMF, reacted at room temperature for 2 hours, and sequentially washed with DMF, MeOH and DMF.
2) Fmoc deprotection
   20% piperidine in DMF was added to the resulting product, a reaction was performed twice for 5 minutes at room temperature, and then washing was sequentially performed with DMF, MeOH, and DMF.
3) The reactions 1) and 2) were repeated to form a peptide backbone (NH₂-E(OtBu)-A-R(Pbf)-P-A-L-L-T(tBu)-S(tBu)-R(Pbf)L-R(Pbf)-F-I-P-K(Boc)-2-chlorotrityl resin).
4) Cleavage: The peptide was isolated from the resin by adding a cleavage cocktail to the synthesis-completed peptide resin.
5) After cooled diethyl ether was added to the obtained mixture, the obtained peptide by centrifugation was precipitated.
6) After purification through Prep-HPLC, a molecular weight was determined by LC/MS and lyophilization was carried out, thereby preparing a powder.

### Example 2: Experiment preparation and materials

### Example 2-1: Culture and treatment of NSCs and primary cortical nerve cells

All procedures involving animals were performed according to the guidelines of the Hanyang University Institutional Animal Care and Use Committee. All experiments were performed with the use of the smallest number of animals and minimized pain thereof, and all animals were only used once in the experiments.

NSCs were isolated from the brain of a rat embryo, cultured and developed. The NSC culture was performed in the same manner as to be described below. Briefly describing, a rat embryo was obtained on day 13 of the embryonic period, and the brain was quickly removed and placed in a petri dish containing cold phosphate-buffered physiological saline (Hank's balanced salt solution; HBSS).

Single cells were isolated from the cerebral cortex, the lateral ganglion eminence, and the embryonic midbrain. These cells were put into a dish pre-treated with L-ornithine and fibronectin, containing calcium and magnesium-free phosphate-buffered physiological saline (PBS) at a density of 20,000 cells/cm². In addition, the cells were cultured in an N2 medium (DMEM F/12, 4.4 nM insulin, 100 mg/L transferrin, 30 nM selenite, 0.6M putrescine, 20 nM progesterone, 0.2 mM ascorbic acid, 2 nM L-glutamine, 8.6 mM D(+) glucose, and 20 nM sodium hydrogen carbonate). The culture was maintained in humidified and 5% CO₂ conditions for 4 to 6 days at 37°C.

NSC differentiation was induced by changing the cell culture medium to a bFGF (10 ng/ml)-free N2 medium, and the medium was exchanged daily.

NSCs were divided into 6 samples, and each sample was treated with 0, 2.5, 5, 20, or 40 µM Aβ25-30 oligomers for 48 hours to identify a suitable concentration. The water-soluble oligomer form of Aβ25-30 was prepared as suggested in the well-known report as follows. Briefly, Aβ25-30 was dissolved in hexafluoroisopropanol to reach a concentration of 1 mM. In addition, the prepared solution was dispensed using a sterilized ultramicrocentrifuge tube. The hexafluoroisopropanol was removed using a vacuum dryer system, and a peptide film was stored and dried at - 20°C. To prepare an oligomer, the peptide was mixed with dried dimethyl sulfoxide to reach a concentration of 5 mM. In addition, the resulting peptide was added to a Ham's F-12 medium to become a final concentration of 1 mM. As described above, the cells were cultured at 4°C for 24 hours.

To identify the PEP1 effect in NSCs, PEP1 was treated at various concentrations of 0, 1, 10, 50, 100, and 200 µM for 48 hours. To identify the PEP1 effect on Aβ25-30-induced neurotoxicity, NSCs were treated with PEP1 at various concentrations of 0, 1, 10, 50 and 100 µM and 20 µM Aβ25-30 for 48 hours. The plate was gently washed with PBS several times, and cell viability was measured using a cell counting kit-8 (CCK-8), MTT and lactate dehydrogenase (LDH).

To identify the role of the PI3K/AKT signaling system in the PEP1 mechanism, NSCs were treated with a PI3K inhibitor LY294002 for 1 hour before treatment of 50 µM PEP1 and 20 µM Aβ25-30. NSCs were divided into four groups as follows: control group (Group 1), group treated with 20 µM Aβ25-35 for 48 hours (Group 2), group treated with 20 µM Aβ25-35, as well as 50 µM PEP1, for 48 hours (Group 3), and group treated with 10 µM LY294002 and 20 µM Aβ25-35, as well as50 µM PEP1, for 48 hours (Group 4).

A primary cultured cortical nerve was obtained from the cerebral cortex of the fetal rat on day 16 of the embryonic period. Briefly, the brain was rapidly removed from the rat, and placed in a petri dish containing cold phosphate-buffered physiological saline (HBSS). Single cells were isolated from the cerebral cortex, and added into a previously poly-L-lysine-coated Corning dish with a diameter of 100 mm, or a glass coverslip was placed in a 6- or 24-well plate. The cultured cells were cultured in DMEM (high glucose).

Two days after the cells were added, non-neuronal cells were removed through treatment of 5 µM cytosine arabinoside for 24 hours. Only the mature culture (7 days in the test tube) was used in this experiment. Approximately 79.7% of a group of neurons was obtained from the neurons of a primary cortex.

Human NSCs (H9 hESC-derived, GIBCO) were plated in a culture dish previously coated with CELLStart (GIBCO) in which Ca2+/Mg2+ is dissolved in D-PBS at a density of 5x10⁴ cells/cm², and cultured in StemPro NSC SFM (KnockOut DMEM/F-12, 2 mM GlutaMAX supplement, 2% StemPro neural supplement, 20 ng/mL basic FGF recombinant protein, and 20 ng/mL EGF recombinant protein). The culture medium was maintained in a humidified 5% CO₂ atmosphere at 37°C for 4 to 6 days.

### Example 2-2: CCK-8 and LDH secretion assay for evaluating cell viability

CCK-8 was used as a method for counting living cells by bonding 2-[2-methoxy-4-nitrophenyl]-3-[4-nitrophenyl]-5-[2,4-disulfophenyl]-2H-tetrazolium, monosodium salt (WST-8) with 1-methoxyphenazinium methylsulfate (PMS). Briefly, cells contained in a 96-well plate were cultured by adding 10 µL of a kit reagent for 3 hours. Cell viability was evaluated by measuring absorbance at 450 nm. All results were normalized based on a cell-free well. Colorimetry was used to measure an LDH level in the cultured NSCs. Cell viability was evaluated by measuring absorbance at 490 nm and reference absorbance at 690 nm. All results were normalized based on a cell-free well.

### Example 2-3: Assay for measuring cell division using bromodeoxyuridine

NSCs were treated with different concentrations of PEP1 and 20 µM Aβ25-30 for 48 hours. Afterward, NSCs were cultured in bromodeoxyuridine (BrdU)-labeled media for 5 hours and observed using a kit. Cell division was evaluated by measuring absorbance at 370 nm and reference absorbance at 492 nm. All results were normalized based on a cell-free well.

### Example 2-4: Colony forming unit (CFU) assay

A CFU assay was carried out by the following method. Briefly, 0.25x10⁵ NSCs were plated in a 6-well plate, and treated with 20 µM Aβ25-30 and then with PEP1 at 1, 10 or 50 µM for 48 hours. The cells were washed with DPBS, and then the medium was exchanged with a fresh cell culture medium. Fourteen days later, the cells were washed again with DPBS, and stained with 0.5% crystal violet dissolved in methanol for 30 minutes at room temperature. Following staining, the plate was washed with DPBS and then dried. The number of colonies was observed using a stereoscopic microscope, and among these colonies, those smaller than 2 mm or faintly stained were removed.

### Example 2-5: TUNEL staining for observing apoptosis

NSCs were placed onto collagen-coated glass coverslips with a diameter of 13 mm, and then treated in the manner described below: 1) treated with Aβ25-30 and PEP1; 2) treated with only 20 µM Aβ25-30; or 3) treated with 20 µM Aβ25-30 and PEP1 at each concentration (1, 10, 50, or 100 µM) for 48 hours.

The cells were washed with PBS and fixed with 4% paraformaldehyde for 20 minutes.

TUNEL staining is a method used to confirm apoptosis. To observe an intact, condensed or fragmented nucleus, the cells that have been stained with TUNEL were treated with 100 µg/ml DAPI for 20 minutes to stain the nucleus, and then mounted on a glass slide to which a DAPI-mixed mounting medium was applied.

The cells were observed under a fluorescence microscope and observed at excitation fluorescence wavelengths corresponding to DAPI and TUNEL.

### Example 2-6: Cell migration assay

A cell migration assay was carried out using a QCM-24-well cell migration kit. Briefly, 20 µM Aβ25-30 and each concentration of PEP1 (1, 10 or 50 µM) were added to a lower chamber, and 3x10⁵ NSCs were cultured in an upper chamber at 37°C for 24 hours. NSCs migrated toward the membrane, and after staining to observe the number of migrating cells, absorbance was measured at 560 nm.

### Example 2-7: Confirmation of penetration of PEP1 into NSCs

NSCs were added at a density of 1x10⁵ cells/well. The cells were exposed to 20 µM Aβ25-30 and treated with 1 µM of FITC-labeled PEP1, as well as a control group, for 0 to 24 hours, thereby enabling the observation that the mitochondria and the cell membrane were stained. The mitochondrial staining was carried out with 100 nM of MitoTracker Red FM at 37°C for 10 minutes, and the cell membrane staining was carried out with 1µM of CellTracker CM-Dil at 37°C for 5 minutes. After staining, the cells were washed with a cell culture medium, and fixed with 2% paraformaldehyde at room temperature for 15 minutes. Afterward, the cells were washed with PBS several times, and the sample was mounted on a glass slide to which a DAPI-containing mounting medium was applied. The cells were observed under a fluorescence microscope using a suitable excitation wavelength.

### Example 2-8: Observation of reactive oxygen species (ROS) production

NSCs were treated with 20 µM Aβ25-30 for 48 hours and stained with DCFH-DA for 15 minutes to measure ROS. NSCs were treated with a varying concentration of PEP1 and 20 µM Aβ25-30 for 48 hours. After culturing, the cells were stained with 10 µM DCFH-DA at 37°C for 15 minutes and washed with PBS three times. DCFH-DA freely passed through the cell membrane, and then was degraded into DCF by an esterase present in the cells. Therefore, a DCF fluorescent intensity refers to an intracellular hydrogen peroxide (ROS) level. DCF accumulation was evaluated using a reader according to an absorbance at 490 nm and a reference absorbance at 690 nm. Flow cytometry was observed and analyzed using an Accuri C6 flow cytometer.

To investigate how Aβ25-30 and PEP1 influenced ROS, a DHBA level was measured and observed by a cell-free method. This method was carried out by observing the DHBA level in a tube containing 20 µM Aβ25-30 and each concentration of PEP1 (0, 1, 10 or 50 µM) through HPLC.

### Example 2-9: Method for observing oxidative DNA damage in mitochondria

The oxidative DNA damage in the mitochondria was observed using an 8-OHdG ELISA kit. The mitochondria DNA was obtained using a genomic DNA isolation kit. 8-OHdG monoclonal antibodies were added to samples and a control group, and then corresponding HRP-attached secondary antibodies were added thereto. The samples were quantified using 8-OHdG and observed by measuring absorbance at 450 nm.

### Example 2-10: Method for measuring mitochondria cell membrane potential

NSCs were plated in a 96-well plate at a density of 5x10⁵ cells/well. The cells were treated with 20 µM Aβ25-30 and each concentration of PEP1 (1, 10 or 50 µM) at 37°C for 48 hours.

The mitochondria cell membrane potential was measured using a JC-1 mitochondria cell membrane potential observation kit. The mitochondria cell membrane potential was observed using an ELISA reader. Healthy cells were observed at a rhodamine wavelength and a Texas red wavelength, and it was confirmed that most JC-1s were aggregated. By observing dead cells or unhealthy cells at an FITC wavelength, it was confirmed that JC-1 is in a monomer state.

### Example 2-11: Adenosine triphosphate (ATP) assay

An ATP concentration was observed using an ATP determination kit and evaluated by an ELISA reader. The ATP concentration was observed at excitation/emission wavelengths of 535/587 nm.

### Example 2-12: Proteomics

### Preparation of protein samples

The cultured NSCs were washed with cold PBS twice, and disrupted in a sample lysis buffer using a sonicator for 10 seconds. In addition, the cells were disrupted again for a further 1 hour at room temperature. After centrifugation at 15°C and 15000 g for 1 hour, a non-water-soluble sample was eliminated, and only water-soluble samples were subjected to two-dimensional electrophoresis. A protein concentration was quantified by a Bradford method.

### Two-dimensional polyacrylamide electrophoresis

200 µg of a protein sample was loaded on an Immobiline DryStrip gel. Electron focusing was performed at 20°C. For electron focusing, a voltage was increased from 150 volts to 3500 volts over 3 hours to allow the sample to enter the gel. Before entering two-dimensionally, the sample was cultured in an equilibration buffer for 10 minutes. The sample contained in the equilibration buffer was permeated into an SDS-PAGE gel, observed using a 2D hopper system under conditions of 20°C and 1700 particles.

### Image analysis

For quantitative analysis, images were analyzed with a PDQuest system. To measure each spot volume, normalization was performed using the total volume of spots. Protein spots that were increased in normalized volume two-fold or higher were selected.

### Peptide mass fingerprinting

To identify a protein through peptide mass fingerprinting, a protein spot was cleaved into smaller peptides using trypsin, well mixed with α-cyano-4-hydroxycinnamic acid in 50% acetonitrile/0.1% TFA, and then analyzed with MALDI-TOF.

Spectra were obtained from 300 shots per spectrum and calibrated using an automatic trypsin digestion peak. A peak list was calibrated using a Flex analysis system. Peak detection was performed by using a threshold as described below. For S/N, the minimum resolution of monoisotopic mass was selected. A MASCOT analysis program was used to identify the protein found by mass fingerprinting. Parameters used to search databases are as follows: enzyme (trypsin); maximum number of missed cleavages; fixed modifications (Cys); variable modifications (Met); monoisotopic mass; mass tolerance.

Peptide mass fingerprinting data was categorized according to probable protein scores.

### Example 2-13: Antibody microarray

For antibody microarray analysis, a panorama antibody cell signaling kit was used. Briefly, 1.5x10⁷ cells were plated in a 100 mm dish and treated with 20 µM Aβ25-35 and 10 µM PEP1 for 48 hours. The cells were harvested to prepare protein cells. Protein samples were labeled with Cy3 and Cy5 and subjected to antibody microarrays. Array slides were read using a scanner, and results from the obtained scans were analyzed using an analysis program.

### Example 2-14: Western blotting and immunostaining

For western blotting, the following antibodies were used: Ki67 (1:200 Abcam), P85a (1:1000, Millipore), pAKT (ser 473, 1:500, Cell Signaling Technology), GSK-3b (ser 9, 1:1000. Santa Cruz Biotechnology), HSTF-1 (1:1000, Santa Cruz Biotechnology), anti-B cell lymphoma-2 (1:1000, Cell Signaling Technology), HMGB-1 (1:500, Cell Signaling Technology), Bax (1:1000, Cell Signaling Technology), Cytosolic cytochrome C (1:200, Cell Signaling Technology), Cleaved caspase 3 (Asp175, 1: 1000, Cell Signaling Technology), c-Myc (1: 1000, Cell Signaling Technology), GFP (1: 1000, Cell Signaling Technology), hTERT (1:500, Santa Cruz Biotechnology), and rTERT (1:500, Santa Cruz Biotechnology).

To confirm an intracellular signaling system, western blotting was performed after 48-hour treatment.

To obtain the mitochondria and a cytoplasmic protein, mitochondria/intracellular protein isolation kits were used (Abcam). All blots were observed using an ECL solution, and observed images were analyzed using an analysis program.

Immunostaining was carried out to confirm intracellular expression and locations. For immunostaining, the following antibodies were used: Ki67 (1:100 Abcam), rabbit anti-doublecortin (1 mg/mL; Abcam), rabbit anti-glial fibrillary acidic protein (GFAP) (1:5000; Abcam), rabbit anti-Tuj1 (1 mg/mL; Abcam), mouse anti-GalC (1 mg/mL; Millipore), and rabbit anti-HMGBl (1:100; Cell Signaling Technology).

Data was observed using an Olympus fluorescence microscope, and suitable fluorescence was used.

### Example 2-15: Detection using Annexin-5 and propidium iodide (experimental method for detecting apoptosis)

Annexin-5 and propidium iodide detection was carried out using an FITC annexin 5 apoptosis kit. Briefly, the harvested cerebral cortex was treated with 20 µM Aβ25-35 and each concentration of PEP1 (1, 10 or 50 µM), and cultured at 37°C for 48 hours. Cells were washed with cold PBS twice, and mixed with a 1x binding buffer. Afterward, the cells were put into a 1.5 ml tube at a density of 1x10⁵ cells/100 µL, and 5 µL of FITC-labeled annexin-5 and 10 µL of propidium iodide were added thereto. The cells were cultured at room temperature for 15 minutes, and after addition of 400 µL of a binding buffer, the cells were analyzed using a flow cytometer.

### Example 2-16: Overexpression of TERT

### Plasmid vector

A hTERT-GFP vector and a shuttle vector were purchased from OriGene Technologies Inc.

### Transfection of Neon genes

Human stem cells (H9 hESCs, GIBCO) were prepared for transfection. The human stem cells were isolated into single cells, and mixed with 20µL of Neon Buffer-R. To perform electroporation in which a DC high voltage was applied to attempt to transfect a gene, cells and 1 µg of TERT were put into a sterilized tube. After a Neon tip was put into a Neon pipette, a mixture of DNA and cells was pipetted into the tip without bubbles. The neon pipette was inserted into a Neon tube containing 3 mL of Neon electrophoresis buffer E. An electric shock of 1300 volts was applied once. After the electric shock, the cells were rapidly transferred to a CellStar-coated culture plate in StemPro NSC SFM.

### Example 2-17: Statistical Analysis

All data was analyzed by conducting each experiment 5 times for statistical analysis, and when the P-value was less than 0.05, it was determined as a significant experimental value.

### Example 3: Analysis of effects of PEP1 on prevention of neuronal damage and regeneration of neurons at cell level

### Example 3-1: Evaluation of influence of Aβ25-35 and PEP1 on survival ability, proliferation and migration of NSCs

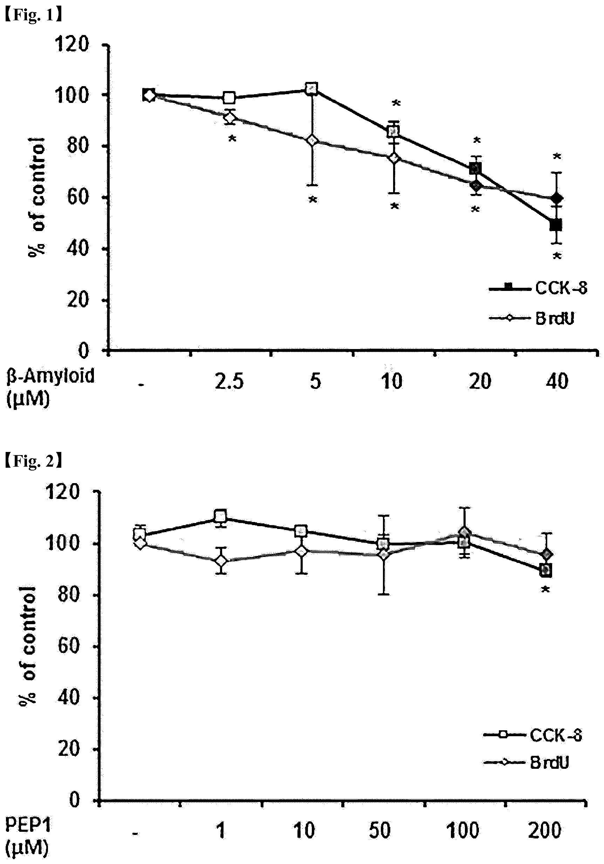

The inventors confirmed whether treatment of different concentrations of Aβ25-35 (0, 2.5, 5, 10, 20, or 40 µM) for 48 hours affected the survival ability, proliferation and migration of NSCs. Cell viability was measured by CCK-8, MIT and LDH assays. Moreover, the proliferation of NSCs was evaluated by BrdU and CFU assays. After treatment with Aβ25-35, cell survival ability and proliferation were significantly reduced in a concentration-dependent method. However, proliferation was reduced at a lower concentration of Aβ25-35, compared to survival ability (refer to FIG. 1).

The inventors treated NSCs with a different concentration of PEP1 (0, 1, 10, 50, 100, or 200 µM) to study the influence of PEP1 on NSCs. Cell survival ability and proliferation were not influenced at a concentration of up to 100 µM, as measured by CCK-8 and BrdU assays (refer to FIG. 2).

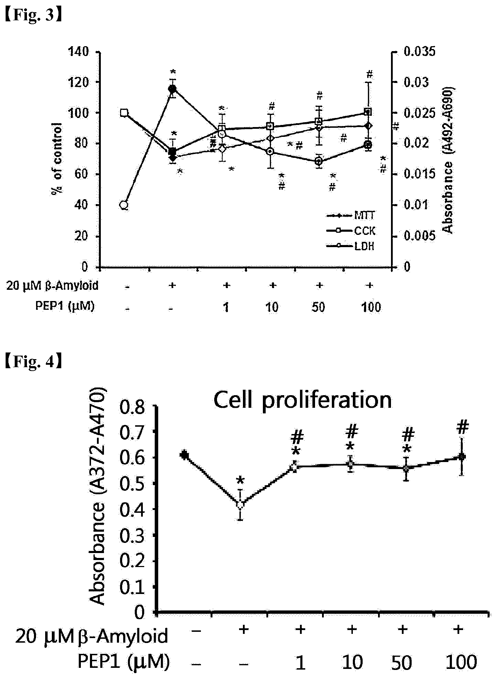

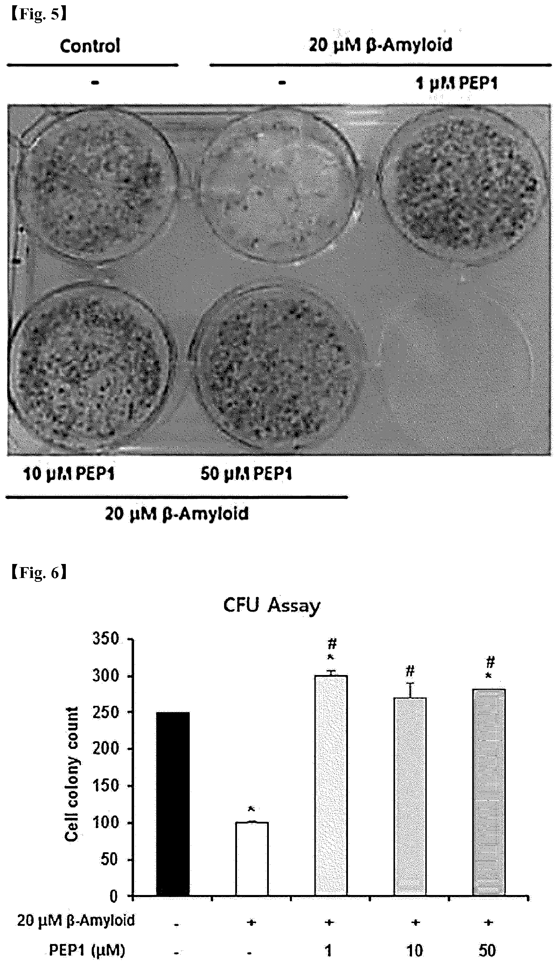

To evaluate the effect of PEP1 on NSCs under an Aβ-induced stress environment, NSCs were exposed to 20 µM Aβ25-35 for 48 hours, and also treated with varying concentrations of PEP1 (0, 1, 10, 50, and 100 µM). The survival ability of NSCs was measured by MTT, CCK-8 and LDH analyses, and proliferation was measured by BrdU and CFU assays. Compared to NSCs only treated with 20 µM Aβ25-35, co-treatment of 20 µM Aβ25-35 and PEP1 (up to 100 µM) gradually increased the survival ability and proliferation of NSCs in a concentration-dependent manner (refer to FIGS. 3 to 6).

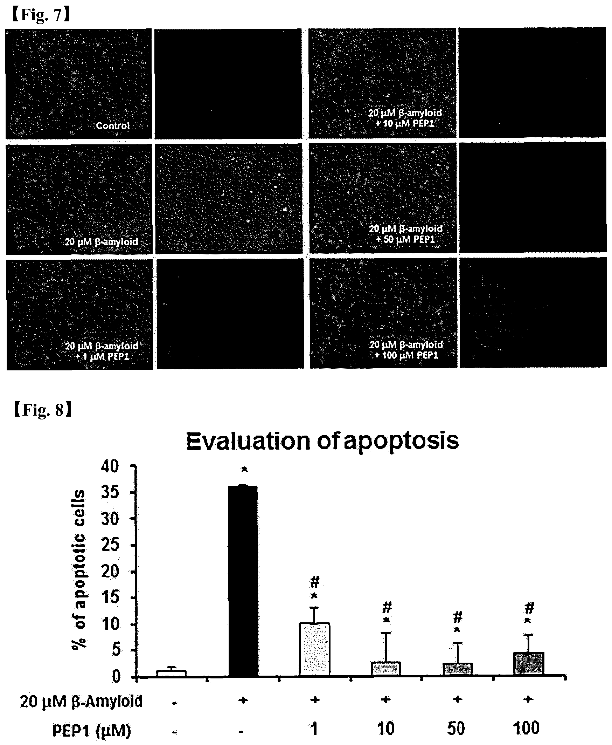

The influence of PEP1 on apoptosis of Aβ-induced NSCs was measured using DAPI and TUNEL staining. A degree of apoptosis of NSCs represented as percentage was significantly increased when the cells were treated with 20 µM Aβ25-35 for 48 hours (p<0.01), and significantly decreased by co-treatment of PEP1 (up to 100 µM) (p<0.05) (refer to FIGS. 7 and 8; the bright part of FIG. 7 is TUNEL-stained NSCs).

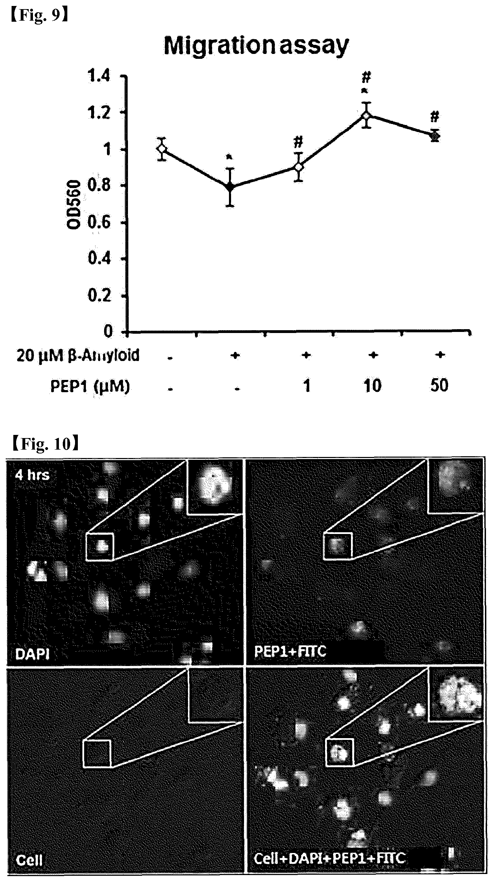

To investigate whether stem cell migration ability, which is a major characteristic of stem cells, is influenced by Aβ and PEP1, NSCs were treated with 20 µM Aβ25-35 and varying concentrations of PEP1 (0, 1, 10, and 50 µM) for 48 hours. Subsequently, migration ability was evaluated using a migration analysis kit. Treatment of 20 µM Aβ25-35 reduced the migration ability of NSCs, but treatment of various concentrations of PEP1 effectively restored migration (refer to FIG. 9).

### Example 3-2: Penetration of PEP1 into NSCs

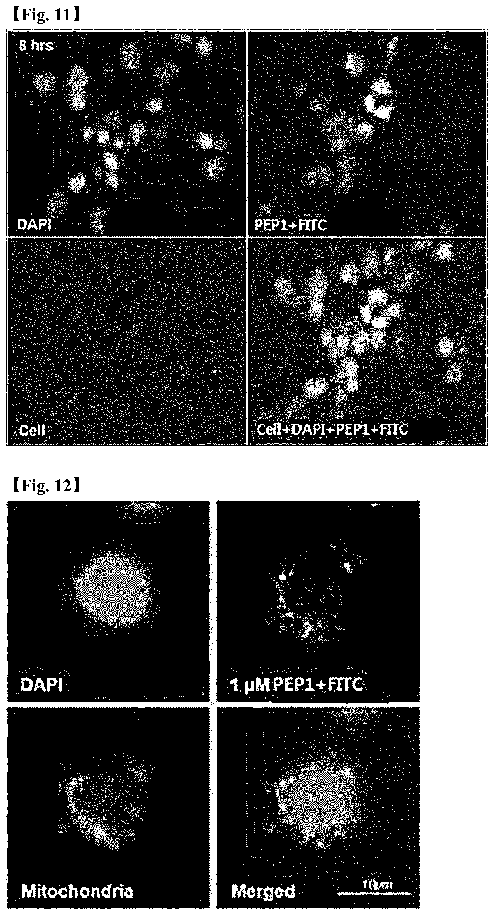

To evaluate the ability of PEP1 to penetrate into NSCs, NSCs were treated with 10 µM of FITC-labeled PEP1 and/or 20 µM Aβ25-35. As a result of the experiment, PEP1 effectively penetrated into NSCs in a normal environment, and usually localized in the cytoplasm and the mitochondria. The PEP1 level in the cells was time-dependently increased. However, when NSCs were damaged by treatment of 20 µM Aβ25-35, it was shown that some of the PEP1 penetrated into the nucleus (refer to FIGS. 10 to 13; the bright parts of FIGS. 10 to 13 are FITC-labeled PEP1).

### Example 3-3: Effect of PEP1 on ROS production and oxidative mitochondrial DNA damage, caused by Aβ25-35

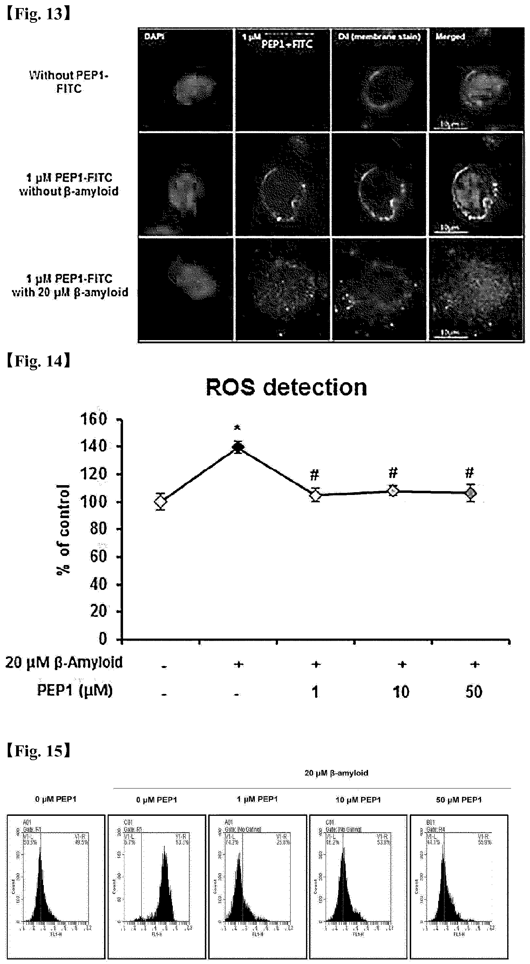

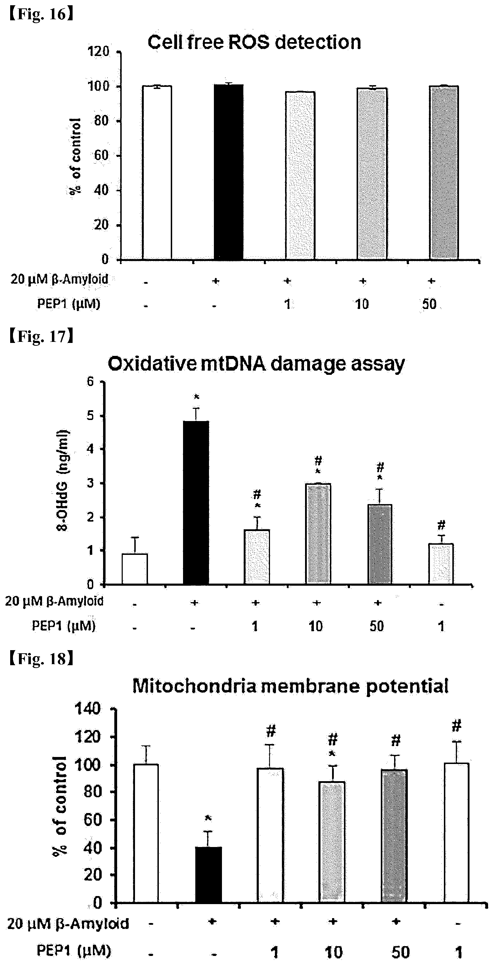

It was examined whether, after the ability of PEP1 to penetrate into cells was observed, PEP1 exhibited an ROS-inhibitory effect. Considering that oxidative stress was induced by Aβ25-35 and apoptosis of NSCs was effectively inhibited by PEP1, in the present invention, it was investigated whether PEP1 has an antioxidant effect. As a result, it was shown that PEP1 significantly reduces an amount of ROS induced by Aβ25-35 (refer to FIG. 14 or 15). In the present invention, as a result of also trying to confirm the direct effect of PEP1 on ROS production, caused by Aβ25-35, but under a cell-free condition, both β-Amyloid and PEP1 did not exhibit a significant influence on ROS production (refer to FIG. 16). Mitochondrial DNA damage caused by oxidative stress was also measured. As a result, it can be seen that the oxidative mitochondrial DNA damage was significantly increased when Aβ25-35 was treated, but effectively decreased when PEP1 was treated (refer to FIG. 17).

### Example 3-4: Influence of PEP1 on mitochondria of NSCs damaged by Aβ25-35

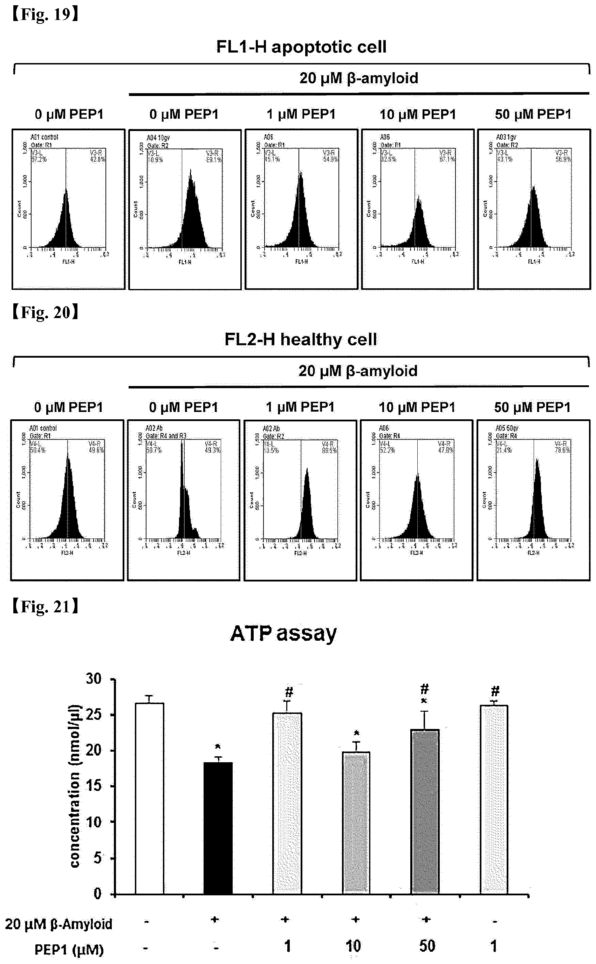

To observe an effect of PEP1 on the mitochondria of NSCs damaged by Aβ25-35 toxicity, the inventors measured mitochondrial cell membrane potential and an ATP level. In NSCs, the mitochondria cell membrane potential was decreased by Aβ25-35, and PEP1 restored the cell membrane potential up to a level in a normal range (refer to FIGS. 18 to 20). The ATP level was decreased by Aβ25-35, and PEP1 significantly restored the ATP level (refer to FIG. 21).

### Example 3-5: Effects of Aβ25-35 and PEP1 on protein level in cells

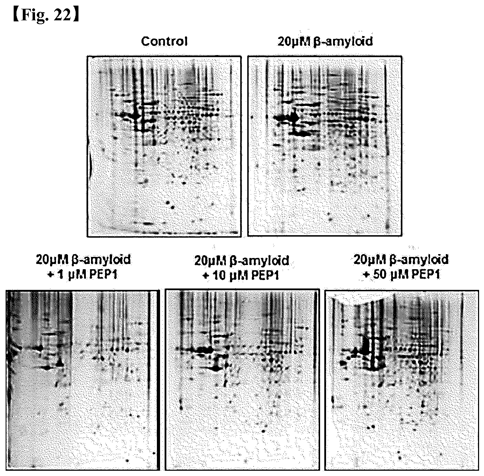

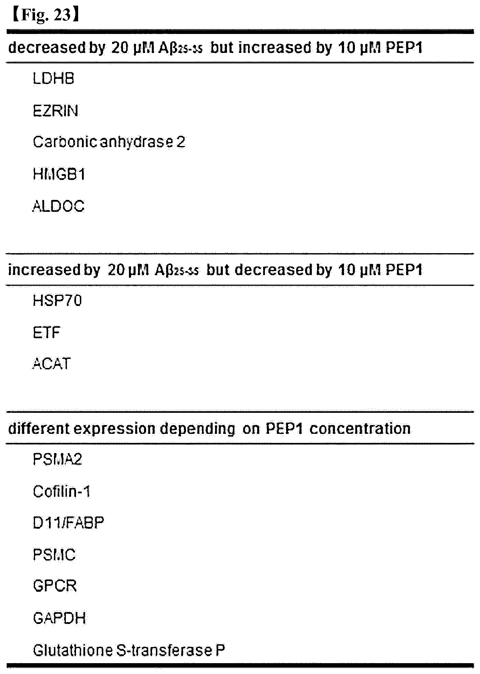

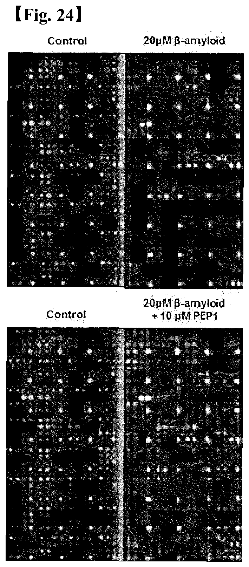

To investigate effects of Aβ25-35 and PEP1 on the production of proteins in NSCs, proteomic studies were conducted. A variety of modified proteins produced by Aβ25-35 and proteins recovered by treatment of PEP1 were quantified. An antibody microarray was performed to observe a phosphorylation pattern of the proteins, and the results thereof showed that various phosphorylated proteins associated with proliferation are decreased by Aβ25-35 and recovered by PEP1 (refer to FIGS. 22 to 25; the bright parts of FIG. 24 are spots associated with inflammation, a TCA cycle, cell migration, and GPCR).

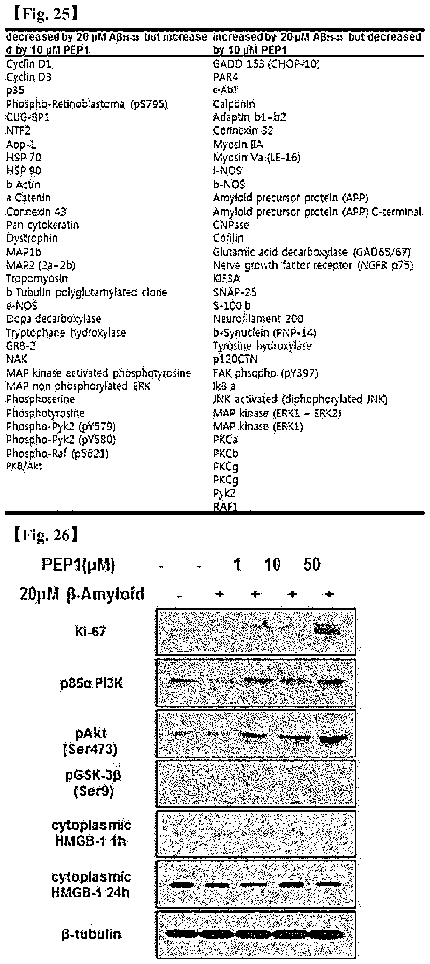

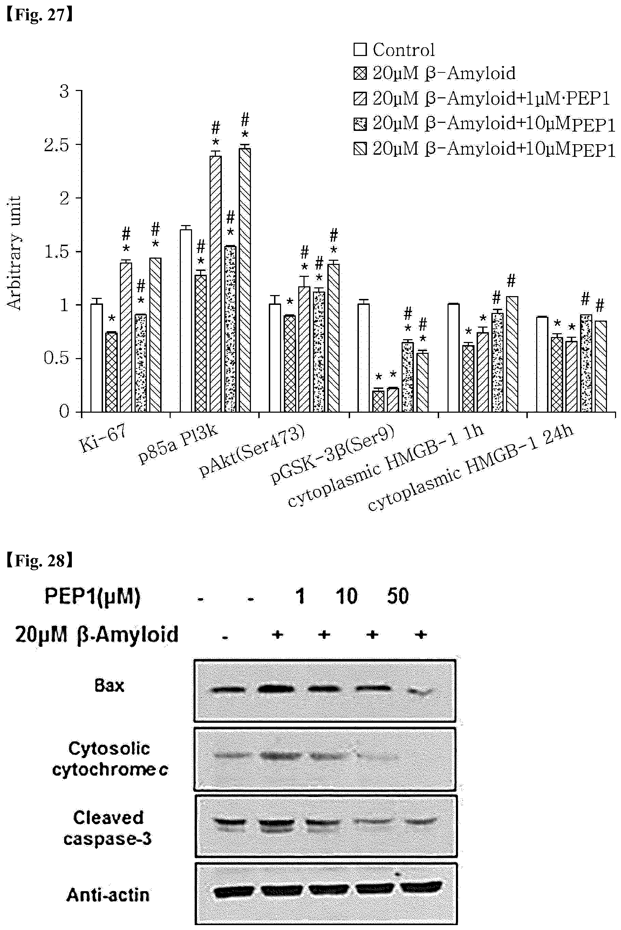

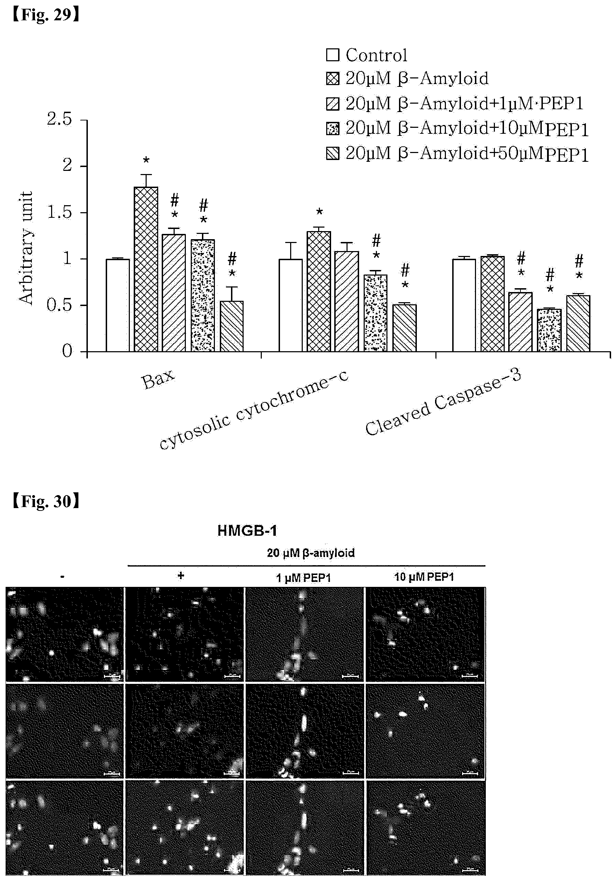

The inventors directly observed immune responses of some proteins induced by Aβ25-35 and PEP1 through western blotting and immunostaining. A protein associated with a proliferation ability in NSCs, ki67, was decreased by Aβ25-35, but concentration-dependently increased by PEP1. Proliferation-associated proteins such as P85a PI3K, and pAKT (Ser 473), phosphorylated GSK3-3b (Ser 9), HSTF-1, and cytoplasmic HMGB-1 were decreased by Aβ25-35, but recovered by PEP1. On the contrary, apoptosis-associated proteins such as Bax, cytoplasmic cytochrome C, and cleaved caspase-3 were increased by Aβ25-35, and significantly decreased by PEP1. HMGB1 expression in the cytoplasm (observed by immunostaining) was reduced by Aβ25-35, but concentration-dependently recovered by co-treatment of PEP1 (refer to FIGS. 26 to 30; the bright parts of FIG. 30 are immunostained parts associated with HMGB-1 expression).

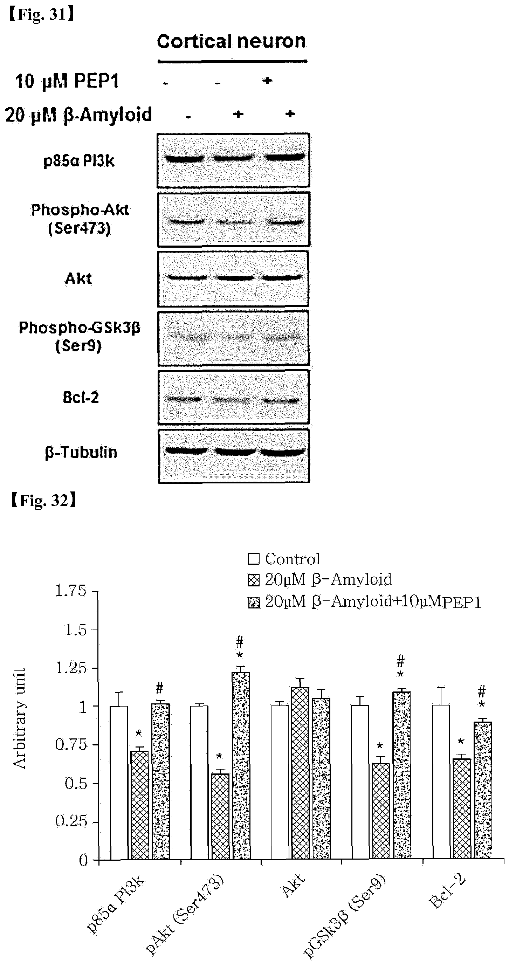

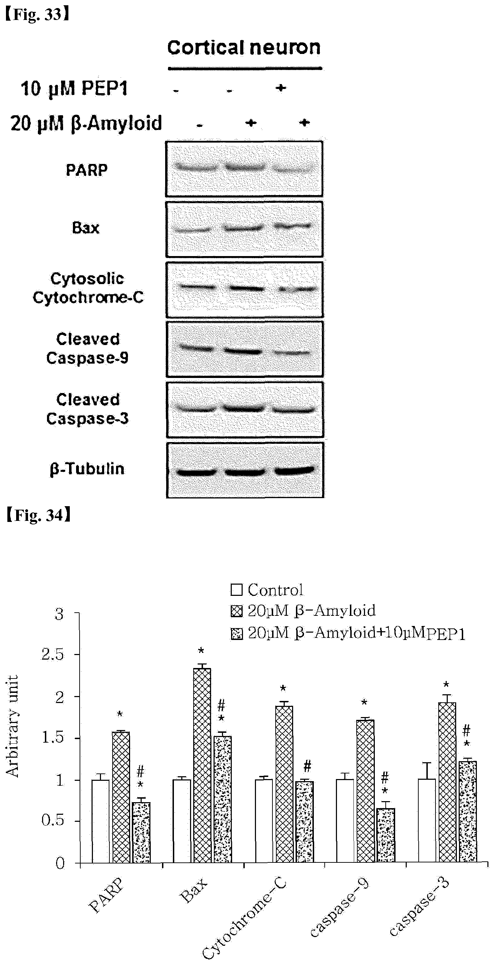

As a result of investigating a PEP1 effect on intracellular signaling proteins in damaged neurons, a large amount of proliferation-associated proteins was observed, and a low amount of apoptosis-associated proteins was observed (refer to FIGS. 31 to 34).

### Example 3-6: Evaluation of PEP1 effect on nerve protection via PI3K pathway induced by Aβ25-35 toxicity

To investigate a PEP1 effect on nerve protection via a PI3K pathway induced by Aβ25-35 toxicity, NSCs were divided into four groups: control group (Group 1), group treated with 20 µM Aβ25-35 for 48 hours (Group 2), group treated with 20 µM Aβ25-35 and 10 µM PEP1 for 48 hours (Group 3), and group treated with 10 µM LY294002 for 49 hours, and then treated with 20 µM Aβ25-35 10 µM PEP1 for 48 hours (Group 4).

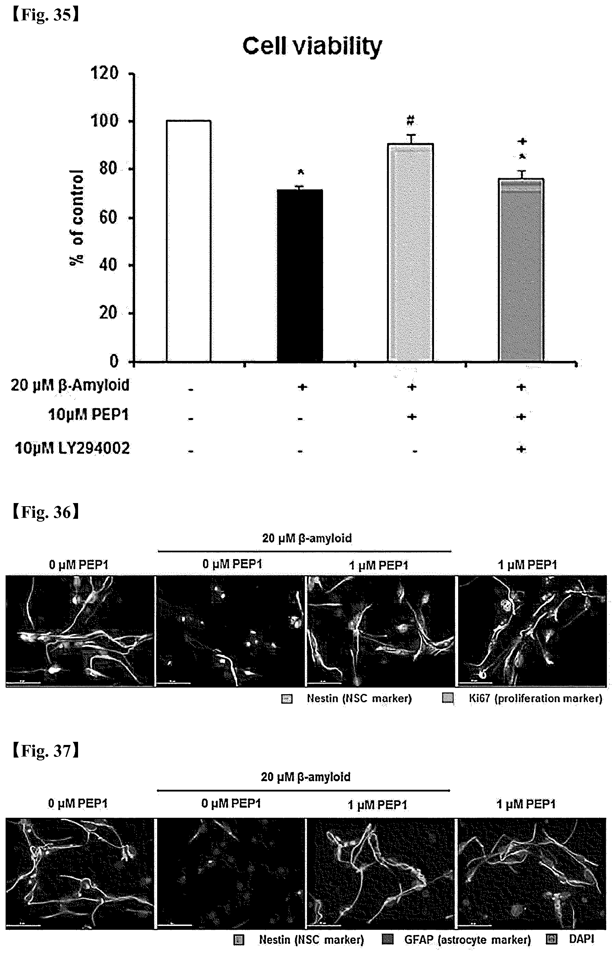

As a result of the experiment, the survival ability of NSCs was degraded approximately 16% in Group 4 pre-treated with a PI3K inhibitor LY294002, compared to Group 3 (refer to FIG. 35), and therefore it was inferred that the PI3K pathway is associated with the nerve protection effect of PEP1.

### Example 3-7: Evaluation of effect of PEP1 on NSC differentiation due to Aβ25-35 toxicity

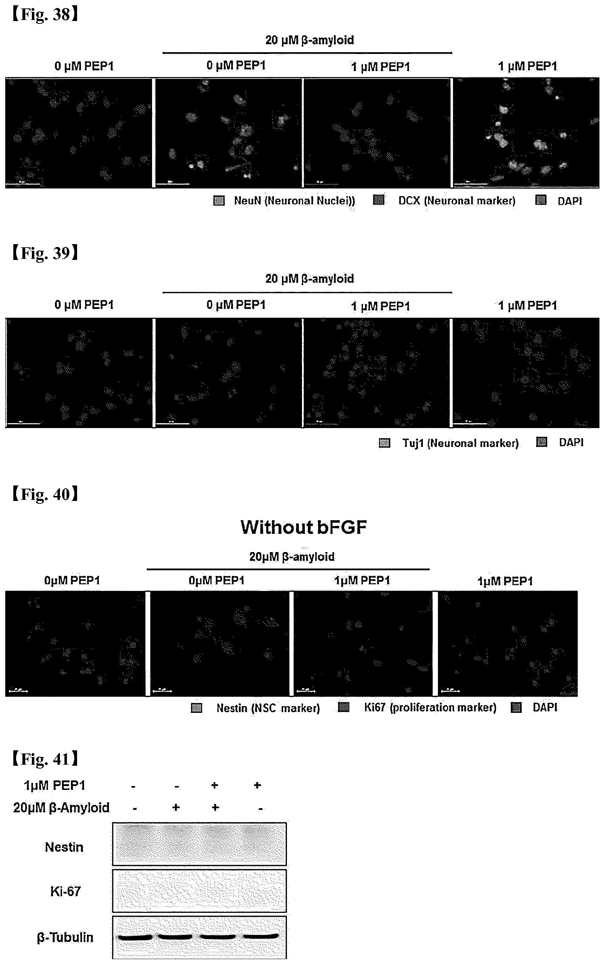

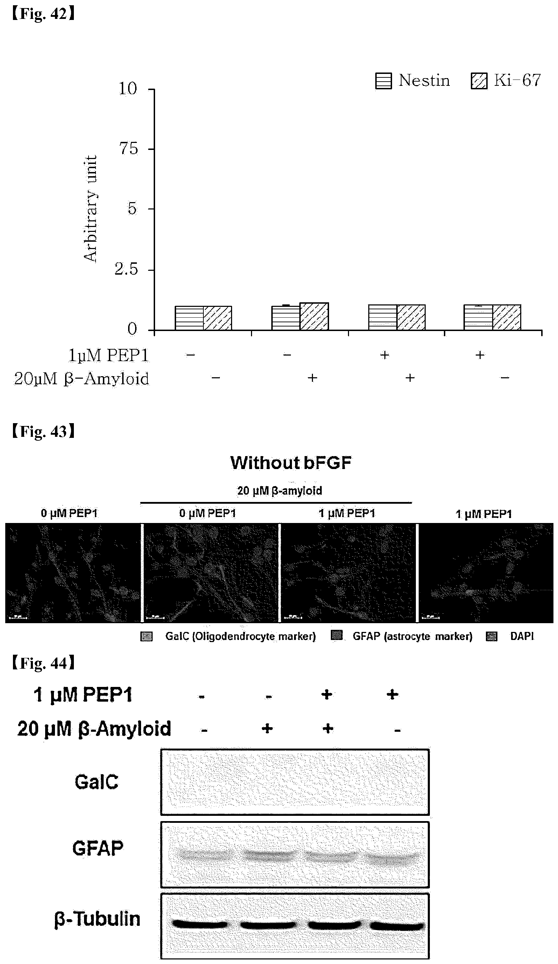

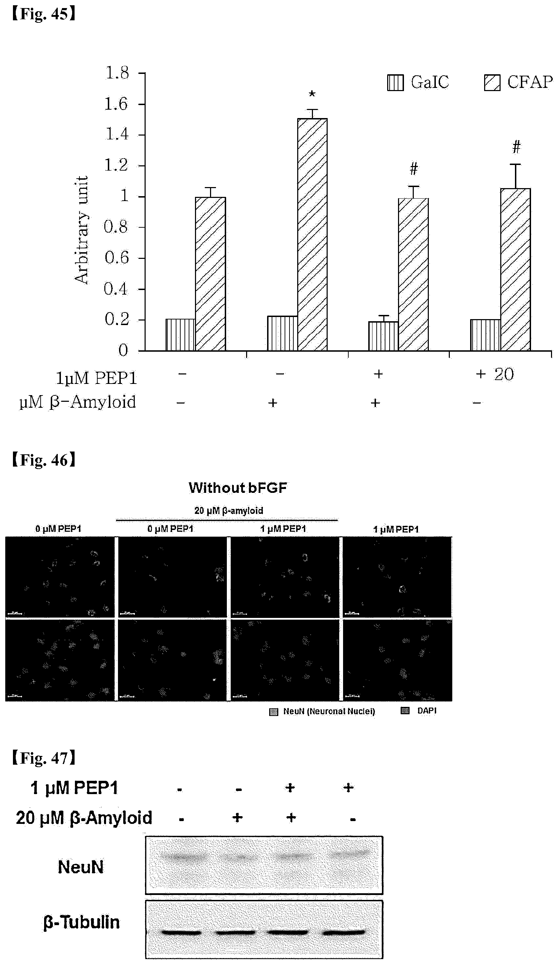

To evaluate the effect of PEP1 on various steps of differentiation of NSCs due to Aβ25-35 toxicity, the inventors of the present invention induced NSC differentiation by changing the culture medium to a bFGF-free N2 medium. Although there was no specificity prior to differentiation, the immune responses of Nestin and Ki67 did not appear after differentiation. However, in a large number of GFAP-positive cells and NeuN-positive cells, such immune responses were increased after differentiation. In addition, the treatment of Aβ25-35 tended to increase NSC differentiation into the GFAP-positive cells and decrease differentiation into the NeuN-positive cells, but the opposite effect was observed in the treatment of PEP1 (refer to FIGS. 36 to 48; the bright part of FIG. 36 is a part that was immunostained to show Nestin or Ki67 expression, the bright part of FIG. 37 is a part that was immunostained to show Nestin, GFAP or DAPI expression, the bright part of FIG. 38 is a part that was immunostained to show NeuN, DCX or DAPI expression, the bright part of FIG. 39 is a part that was immunostained to show Tuj1 or DAPI expression, the bright part of FIG. 40 is a part that was immunostained to show Nestin, Ki67 or DAPI expression, the bright part of FIG. 43 is a part that was immunostained to show GalC, GFAP or DAPI expression, and the bright part of FIG. 46 is a part that was immunostained to show NeuN or DAPI expression).

### Example 3-8: Evaluation of effect of PEP1 on hTERT overexpression in human NSCs

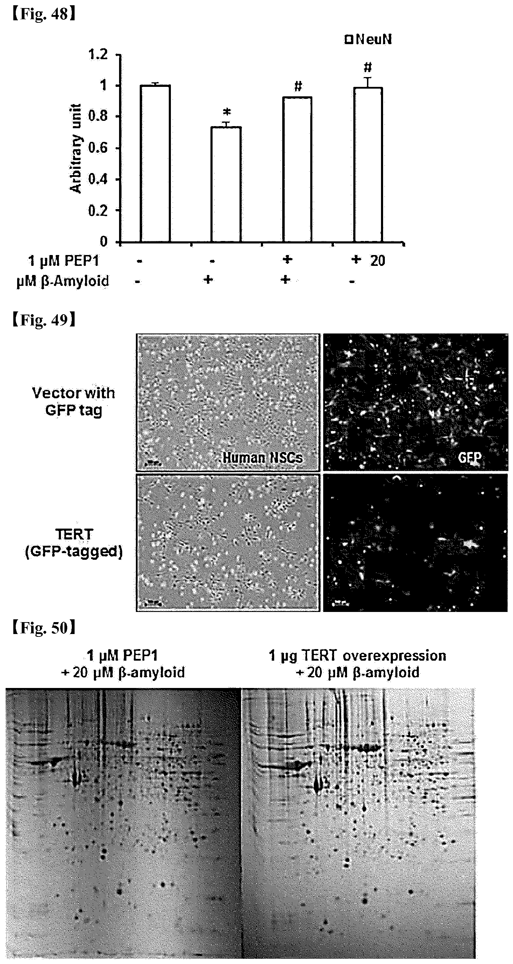

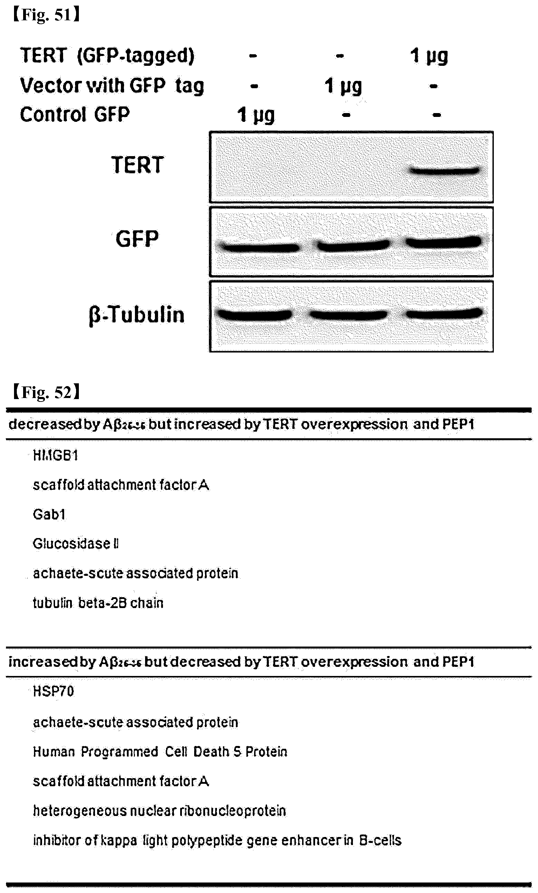

To confirm whether the effect of PEP1 is directly associated with extracellular telomerase activity of hTERT, the inventors induced hTERT overexpression in human NSCs. Despite the hTERT overexpression, the survival ability of the human NSCs was decreased by gene transfection. According to proteomic results, it was shown that similar proteins affected by PEP1 treatment were also affected by the hTERT overexpression (refer to FIGS. 49 to 54).

### Example 4: Analysis of effect of PEP1 on prevention of neuronal loss and regeneration of neurons in animal models (3XTg-AD)

### Example 4-1: Preparation of animal models for experiment and design of experiment

To test the efficacy of PEP1 on AD, AD transgenic mice (3XTg-AD) were purchased from Jackson Laboratory. Genetic characteristics of the transgenic mice are as follows: B6; 129-Psen1tm1Mpm Tg(APPSwe, tauP301L)1Lfa/Mmjax. After the 8-week-old transgenic mice were preliminary-bred, the mice were housed in a cage at a male-female ratio of 1:2 to increase the number of transgenic mice for approximately 7 or 8 days and then separated, and pregnancy of the mice was observed for approximately 7 to 8 weeks. One week after mice were confirmed as pregnant, new mice were delivered and weaned for 3 weeks. Two weeks later, mature mice were obtained.

During the preliminary breeding and the entire period of the experiment, a temperature was maintained at 23±2°C and a relative humidity was maintained at 60±10%, and then the mice were raised under a 12-hour dark/light cycle. Experimental animals were divided into five groups, and each group included 10 animals. The divided experimental groups are as follows. Group 1: treated with physiological saline; Group 2: treated with 0.01 mg/kg of PEP1; Group 3: treated with 1 mg/kg of PEP1; Group 4: treated with 1 mg/kg of donepezil, positive control group; and Group 5: treated with a scramble peptide (random peptide with a length of 16 amino acids).

The PEP1, physiological saline, donepezil and scramble peptide were subcutaneously injected at each concentration three times a week for two months from the age of 12 months, and then two months after the injection, cognitive/memory behavior tests (passive avoidance test and Y maze test) were performed to examine a degree of improvement in cognitive function and memory function. After the brain was extracted to separate the hippocampus, the amounts of amyloid beta in the hippocampus and the whole brain, and the amounts of an amyloid beta oligomer and NFT generated by tau hyperphosphorylation were determined by western blotting and immunostaining. In addition, various changes in specific protein due to PEP1 in the brain were observed through western blotting.

### Example 4-2: Method for analyzing various proteins in hippocampus and whole brain using immunostaining

After anesthesia, the mice that finished the animal behavior test (10 mice per group) were subjected to reperfusion of the heart using 0.9% physiological saline, and then reperfused again with 4% paraformaldehyde for fixation. The 4% para-fixed brain was removed and added into a 15 ml tube containing 4% paraformaldehyde. Next day, the brain maintained as such was added in 30% sucrose. The brain was left until the brain was immersed in 30% sucrose. The fixed tissue was frozen at -20°C using an O.C.T compound. The frozen tissue was cut to a thickness of 20 µm using a cryostat. The cut tissue section was treated with 50% ethanol mixed with 0.01 M PBS for 30 minutes to allow the antibodies to easily permeate through the tissue. The tissue was treated with 0.3% hydrogen peroxide for 10 minutes to remove the activity of an inherent peroxidase in the tissue. The tissue section was left in 10% normal serum (N/S) for one hour. Afterward, the tissue was left in a solution containing primary antibodies diluted to 0.5% N/S for one day at 4°C.

The primary antibodies used for immunofluorescent staining are as follows: anti-β-amyloid (D54D2) (1:100, Cell Signaling, Beverly, MA, USA), anti-oligomer β-amyloid (1:100, Millipore), anti-Tau (2 µg/ml, Invitrogen), anti-NeuN (1:100, Millipore), anti-DCX (1:100, Cell Signaling), anti-Tuj1 (2 µg/ml, Abcam), anti-GFAP (1:1,000, Abcam) and anti-GalC (1:000, Millipore).

Afterward, the tissue section was left in a secondary antibody-containing solution for 2 hours. The secondary antibodies used herein are as follows: goat-anti-rabbit Alexa Fluor 488 (Molecular Probes), anti-rabbit tetramethylrhodamine (Molecular Probes), and goat-anti-mouse Alexa Fluor 488 (Molecular Probes).

The tissue section was washed with PBS three times, placed on a glass slide, and then mounted using a DAPI-containing mounting medium (Vector Laboratories). Afterward, the tissue section was observed using a fluorescence microscope.

### Example 4-3: Evaluation of effect of PEP1 on NeuN expression

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lyzed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF) and 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a neuronal nuclei (NeuN, MAB377, Millipore) antibody solution in 5% BSA (bovine serum albumin) at 1:1000 dilution all day at 4°C. The membrane was washed with 0.1% Tween 20-containing TBS/T and reacted with HRP-conjugated anti-mouse IgG antibodies diluted at 1:3000 for one hour. The membrane was washed with TBS/T and visualized using a West-Q Chemiluminescent Substrate Plus Kit (GenDEPOT, TX, USA), followed by analysis of band intensity of the image using an image analyzer.

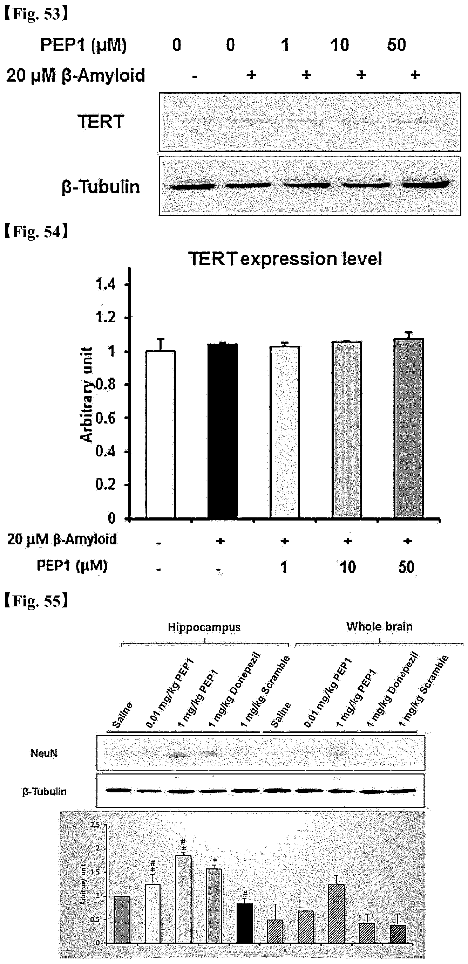

As a result of the analysis, compared to the control group and the positive control group (Donepezil), when the same amount of PEP1 was treated, it was shown that NeuN was increased in the hippocampus and the whole brain (refer to FIG. 55). The presence of a large number of markers for mature neurons, NeuN, which were expressed in the nuclei of post-mitotic neurons, due to PEP1 may indicate that many neurons can be produced due to PEP1.

### Example 4-4: Evaluation of effect of PEP1 on DCX expression

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lyzed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF) and 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a GFAP (ab7260, Abcam) antibody solution in 5% BSA at 1:10000 dilution for all day at 4°C. The membrane was washed with 0.1% Tween-20-containing TBS/T and reacted with HRP-conjugated anti-rabbit IgG antibodies diluted at 1:3000 for 1 hour. The membrane was washed with TBS/T and visualized using a West-Q Chemiluminescent Substrate Plus Kit (GenDEPOT, TX, USA), followed by analysis of band intensity of the image using an image analyzer.

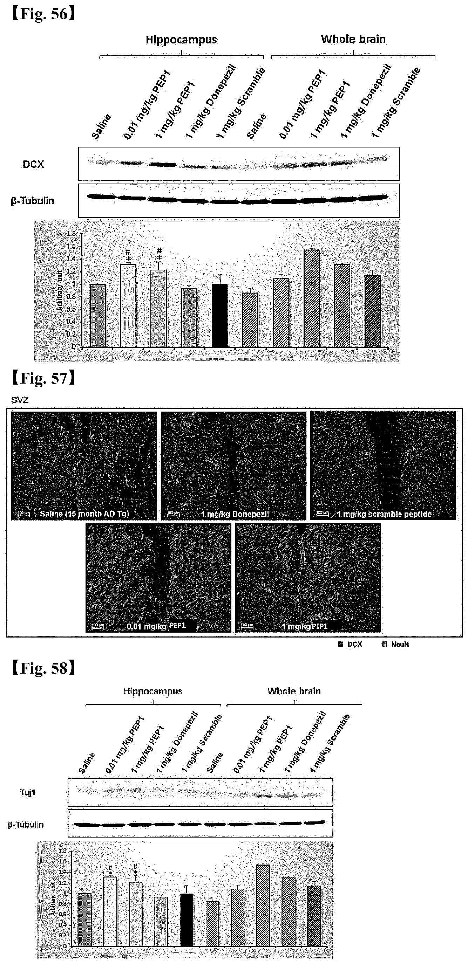

As a result of the analysis, compared to the control group and the positive control group (Donepezil), when the same amount of PEP1 was treated, it was shown that DCX was increased in the hippocampus and the whole brain (refer to FIGS. 56 and 57; the bright part of FIG. 57 is a part that was immunostained to show DCX or NeuN expression). Considering that DCX highly expressed in neural progenitors and immature neurons is highly expressed due to PEP1, it can be seen that early neuronal progenitors are highly expressed due to PEP1, indicating that PEP1 can affect proliferation of neurons.

### Example 4-5: Evaluation of effect of PEP1 on Tuj1 expression

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lyzed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF), and 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a Tuj1 (ab18207, Abcam) antibody solution in 5% BSA at 1:10000 dilution all day at 4°C. The membrane was washed with 0.1% Tween-20-containing TBS/T and reacted with HRP-conjugated anti-rabbit IgG antibodies diluted at 1:3000 for 1 hour. The membrane was washed with TBS/T and visualized using a West-Q Chemiluminescent Substrate Plus Kit (GenDEPOT, TX, USA), followed by analysis of band intensity of the image using an image analyze.

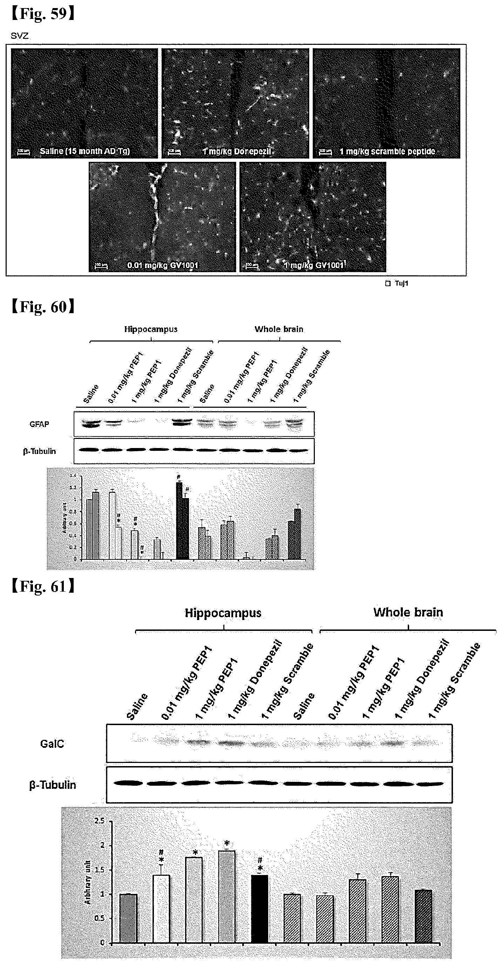

As a result of the analysis, compared to the control group and the positive control group (Donepezil), when the same amount of PEP1 was treated, it was shown that Tuj1 was increased in the hippocampus and the whole brain (refer to FIGS. 58 and 59; the bright part of FIG. 59 is a part that was immunostained to show Tuji expression). Considering that Tuj1 which is known to be expressed in dividing neuronal precursors or newly-produced immature post-mitotic neurons is highly expressed due to PEP1, it can be seen that the PEP1 administration can affect the induction of division, generation and differentiation of new neurons.

### Example 4-6: Evaluation of effect of PEP1 on GFAP expression

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lyzed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF), 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a GFAP (ab7260, Abcam) antibody solution in 5% BSA at 1:10000 dilution for all day at 4°C. The membrane was washed with 0.1% Tween-20-containing TBS/T and reacted with HRP-conjugated anti-rabbit IgG antibodies diluted at 1:3000 for 1 hour. The membrane was washed with TBS/T and visualized using a West-Q Chemiluminescent Substrate Plus Kit (GenDEPOT, TX, USA), followed by analysis of band intensity of the image using an image analyze.

As a result of the analysis, compared to the control group and the positive control group (Donepezil), when the same amount of PEP1 was treated, it was shown that GFAP was reduced in the hippocampus and the whole brain (refer to FIG. 60). GFAP expressed in astrocytes of the central nervous system causes a disease such gliosis when overexpressed or abnormally expressed, and considering that GFAP overexpressed in dementia-induced animal models is reduced by PEP administration, it can be seen that PEP1 has an effect of preventing and reducing the occurrence of a neuronal disease.

### Example 4-7: Evaluation of effect of PEP1 on Gale expression

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lyzed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF), 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a galactocerebroside (GalC, MAB342, Millipore) antibody solution in 5% BSA at 1:500 dilution for all day at 4°C. The membrane was washed with 0.1% Tween-20-containing TBS/T and reacted with HRP-conjugated anti-mouse IgG antibodies diluted at 1:3000 for one hour. The membrane was washed with TBS/T and visualized using a West-Q Chemiluminescent Substrate Plus Kit (GenDEPOT, TX, USA), followed by analysis of band intensity of the image using an image analyze.

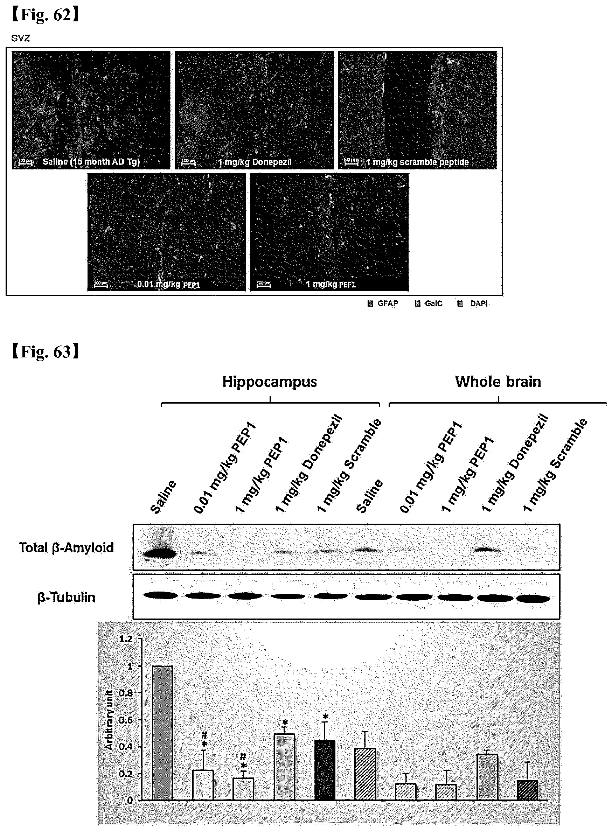

As a result of the analysis, compared to the control group and the positive control group (Donepezil), when the same amount of PEP1 was treated, it was shown that Galc was increased in the hippocampus and the whole brain (refer to FIGS. 61 and 62; the bright part of FIG. 62 is a part that was immunostained to show GFAP, GalC or DAPI expression). Galc is an enzyme that removes galactose and as a marker for oligodendrocytes, indicates that NSCs have differentiated into oligodendrocytes. Considering that PEP1 induces an increase in Galc, it can be seen that PEP1 affects generation of oligodendrocytes necessary for healthy neurons.

### Example 4-8: Evaluation of effect of PEP1 on expression levels of all types of Aβs

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lysed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF), 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in an anti-β-amyloid (D54D2; Cell Signaling, Beverly, MA, USA) antibody solution in 5% BSA at 1:1000 dilution for all day at 4°C. The membrane was washed with 0.1% Tween-20-containing TBS/T and reacted with HRP-conjugated anti-rabbit IgG antibodies diluted at 1:2000 for 1 hour. The membrane was washed with TBS/T and visualized using a West-Q Chemiluminescent Substrate Plus Kit (GenDEPOT, TX, USA), followed by analysis of band intensity of the image using an image analyzer.

As a result of the analysis, compared to the control group and the positive control group (Donepezil), due to the treatment of PEP1, the expression levels of all types of Aβs were decreased in the hippocampus and the whole brain (refer to FIG. 63). It can be shown that PEP1 is formed in an oligomer, thereby reducing the absolute amount of Aβs in a stage before becoming a plaque form, and thus can prevent neuronal loss.

### Example 4-9: Evaluation of effect of PEP1 on expression level of oligomer Aβ

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lysed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF), 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a β-Amyloid oligomer (AB9234, Millipore) antibody solution in 5% BSA at 1:1000 dilution for all day at 4°C. The membrane was washed with 0.1% Tween-20-containing TBS/T and reacted with HRP-conjugated anti-rabbit IgG antibodies diluted at 1:3000 for 1 hour. The membrane was washed with TBS/T and visualized using a West-Q Chemiluminescent Substrate Plus Kit (GenDEPOT, TX, USA), followed by analysis of band intensity of the image using an image analyzer.

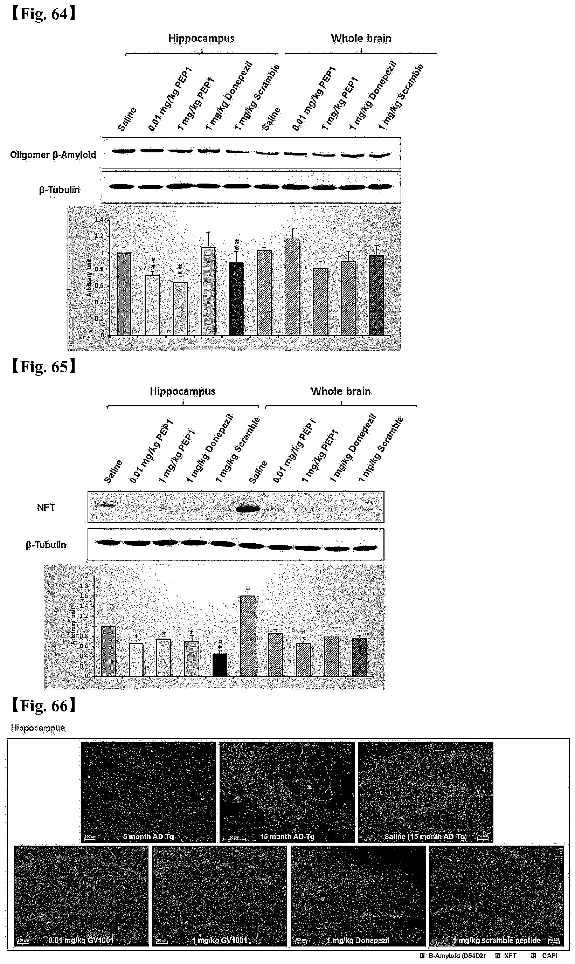

As a result of the analysis, compared to the control group and the positive control group (Donepezil), due to the treatment of PEP1, the expression level of oligomer Aβ was decreased in the hippocampus and the whole brain (refer to FIG. 64). It can be shown that PEP1 is formed in a plaque form, thereby reducing the absolute amount of oligomer Aβs immediately before induction of external damage and death of neurons, and thus can prevent and treat neuronal damage and death.

### Example 4-10: Evaluation of effect of PEP1 on NFT generation

After anesthesia, the mice that finished the animal behavior test (10 mice per group) were subjected to reperfusion of the heart using 0.9% physiological saline, and then whole blood was removed. Afterward, the brain was extracted to divide the hippocampus part and the whole brain part, thereby obtaining brain tissues, and the brain tissues were rapidly frozen using liquid nitrogen. Afterward, the tissues were disrupted, and lysed by adding a lysis buffer (IX RIPA II cell lysis buffer, 1 mM sodium orthovanadate (Na₃VO₄), 100 mg/ml phenyl methyl sulfonyl fluoride (PMSF), 1 mM sodium fluoride (NaF), 1X protease inhibitor cocktail). An amount of the obtained protein was quantified, SDS-PAGE electrophoresis was performed with the same amount (35 µg) of a lysate, and then the protein that was separated through electrophoresis was transferred to a PVDF membrane. The transferred protein-attached PVDF membrane was first left in 2% skim milk for 1 hour, and then left in a solution prepared by diluting 1 µg/ml of Tau (Neurofibrillary Tangles Marker, AHB0042, Invitrogen) antibodies in 5% BSA for all day at 4°C. The membrane was washed with 0.1% Tween-20-containing TBS/T and reacted with HRP-conjugated anti-mouse IgG antibodies diluted at 1:3000 for one hour. The membrane was washed with TBS/T and visualized using a West-Q Chemiluminescent Substrate Plus Kit (GenDEPOT, TX, USA), followed by analysis of band intensity of the image using an image analyzer.

As a result of the analysis, compared to the control group and the positive control group (Donepezil), due to the treatment of PEP1, the expression level of Tau was decreased in the hippocampus and the whole brain (refer to FIGS. 65 and 66; the bright part of FIG. 66 is a part that was immunostained to show β-amyloid, NFT or DAPI expression). A PEP1-induced decrease in Tau expression, which is used to measure the degree of NFT generation causing the damage and death of neurons due to entanglement of inner structures of the neurons, demonstrates that PEP1 prevents the NFT generation, thereby being effective in prevention of neuronal loss.

### Example 5: Evaluation of effect of PEP1 on prevention of neuronal loss and regeneration of neurons according to behavior test analysis for animal models

### Example 5-1: Evaluation of effect of PEP1 in animal models according to passive avoidance test

A passive avoidance test was carried out on ten AD transgenic mice into which a drug was injected as described in Example 4-1 for each group. Two months after the drug injection, the passive avoidance test was performed as follows. A box with a bright room and a dark room was prepared, and a door was made to allow free passage between these rooms. On the first day of the animal test, the mice were put into the room with light, and as soon as they moved, the door toward the room without light was automatically opened for 180 seconds. The following day, the test was carried out in the same manner as the first day, and when the mice entered the room without light, DC electricity of 5V/0.05mA was applied for 10 seconds. On the third day, the test was carried out in the same manner as the first day, and then time and frequency of entry into the room without light were measured. Every waiting time did not exceed 180 seconds. On the third day of the animal behavior test, the mice might not move, and if so, no physical stimulus was given to make them move.

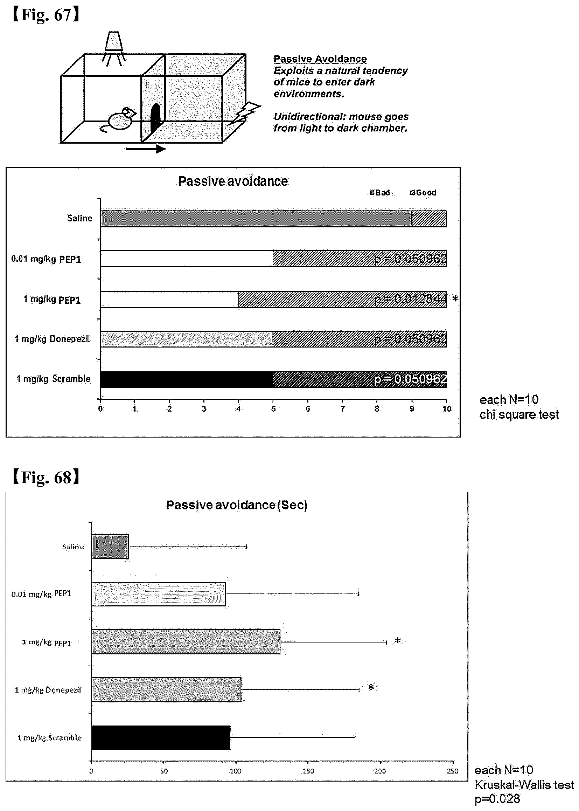

As a result of the analysis, compared to the control group and the positive control group (Donepezil), the PEP 1-treated mice exhibited excellent avoidance ability (refer to FIGS. 67 and 68). It is known that an increase in cognitive ability is influenced by the state of neurons, and according to the test result, it can be presumed that, the treatment of PEP1 is effective in prevention of neuronal loss and regeneration of neurons, and therefore cognitive ability is increased.

### Example 5-2: Evaluation of effect of PEP1 in animal models according to Y-maze test

According to the method described in Example 4-1, a Y-maze test was performed on ten AD transgenic mice into which different drugs had been injected for each group. The Y-maze test is a test for examining whether PEP1 helps spatial perception ability and short-term memory recovery in the AD transgenic mice. A behavior test was performed in a Y-shaped maze with three arms. Each arm was arranged at a predetermined angle of 120° relative to each other. The mice were allowed to freely move between these three arms. The mice seemed to be comfortable exploring new places. Therefore, the mice generally liked go to a new arm, rather than coming back to a visited arm. The arms were defined as A, B and C areas, and the head of a mouse was placed toward the wall of one area to allow the mouse to freely move for 180 seconds, and then the areas where the experimental animals entered were observed and recorded. The number and order of entry into each arm were measured to evaluate spontaneous alteration (%).

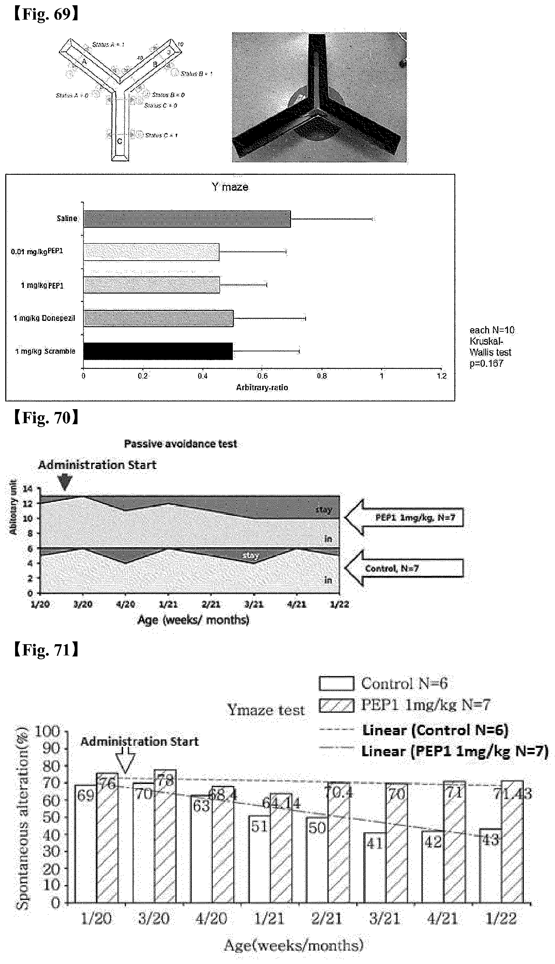

As a result of the analysis, compared to the control group and the positive control group (Donepezil), the animals treated with PEP1 showed excellent behavioral judgment (refer to FIG. 69). The Y-maze test is a test that can determine an ability to select a new path and an ability to remember a path already traveled. According to a test result, considering that the PEP 1-administered animals showed a higher ability, the treatment of PEP1 can improve memory ability and acting power as well as cognitive ability.

### Example 5-3: Evaluation of effect of PEP1 on prevention of neuronal loss and regeneration of neurons according to analysis of behavior test for severe AD animal models

20-month-old or older AD transgenic mice corresponding to severe AD models were prepared by breeding AD transgenic mice (3XTg-AD) for 20 months or longer by the method described in Example 4-1. Two months after PEP1 and physiological saline were subcutaneously injected three times a week for two months from the age of 20 months, cognitive/memory behavior tests (a passive avoidance test and a Y-maze test) were performed to evaluate the degree of enhancement of a cognitive function and a memory function. The cognitive/memory behavior tests were carried out on severe AD transgenic mice, such as seven mice of the PEP1 (1 mg/kg)-administered group and six mice of the physiological saline-administered group (control) by a method similar to the method described in Example 5.

More specifically, two months after the drug administration, a passive avoidance test in which a box with a bright room and a dark room was prepared, the time and frequency of entry into the room without light after electrical stimulation were measured, and a Y-maze test in which mice were allowed to freely move for 180 seconds in a Y-shaped maze with three arms and then the number and order of the entry into each arm were measured to evaluate spontaneous alteration (%) was performed (refer to Examples 5-1 and 5-2 for specific test methods).

As an analysis result, in the case of the passive avoidance test, compared to the control group, the PEP 1-administered group was increased in the time and frequency of staying in the bright room, and thus exhibited excellent avoidance, that is, memory recovery (refer to FIG. 70), and in the case of the Y-maze test, compared to the control group, the PEP1-administered group was increased in ability to select a new path (spontaneous alteration (%)) and thus exhibited an excellent ability to recover cognition/memory (refer to FIG. 71). As described above, it can be seen that the PEP 1-administered models tend to exhibit higher cognitive/memory ability for severe AD, indicating that the mice with severe AD can be improved in cognitive ability, memory ability and acting power by PEP1 administration.

### Example 6: Evaluation of effect of PEP1 on prevention of neuronal loss and regeneration of neurons according to clinical trial

### Example 6-1: Administration method and target for clinical trial

Clinical trials were performed by recruiting subjects with mild, moderate and severe ADs, which are known as the most representative of neuronal loss and neurodegenerative diseases caused thereby. Experiments were designed as a randomized, placebo-controlled, double-blind, parallel designed, multicenter phase 2 clinical trial, and the progression of the disease is determined using a widely known method for measuring the severity of dementia.

In the clinical trial, administration was performed on four groups as follows:
1) Control group: A placebo (0.9% saline) was subcutaneously administered a total of 26 times, for example, 4 times every one week and 22 times every second week.
2) Experimental Group 1: 0.40 mg of PEP1 prepared according to Example 1 was subcutaneously administered a total of 26 times, for example, 4 times every one week and 22 times every second week.
3) Experimental Group 2: 0.56 mg of PEP1 prepared according to Example 1 was subcutaneously administered a total of 26 times, for example, 4 times every one week and 22 times every second week.
4) Experimental Group 3: 1.12mg of PEP1 prepared according to Example 1 was subcutaneously administered a total of 26 times, for example, 4 times every one week and 22 times every second week.

The clinical trial was designed as a randomized, placebo-controlled, double-blind, parallel designed, multicenter phase 2 clinical trial. Subjects suitable for the clinical trial were randomly assigned to one of the three experimental groups and the placebo group. Among the randomly-assigned subjects, one corresponding to the experimental group was subcutaneously administered with a predetermined dose of a drug a total of 26 times, for example, 4 times every one week and 22 times every second week. A subject selected for a control group was treated with the same amount of a placebo (0.9% saline) as a test drug by the same method as described above.

### Example 6-2: Evaluation criteria and method for clinical trial

Analysis of efficacy variables was performed on a full analysis set (FAS) and compared with an additional result obtained with a per-protocol set (PPS).

The efficacy variables were divided into two types.
1) Primary efficacy variables (Primary Endpoints): the variations of ADAS-cog and ADCS-ADL, relative to the baseline (measured at week 0, 12, 24, 36, and 48)
2) Secondary efficacy variables (Secondary Endpoints): the variations of CIBIC-Plus and QOL-AD, relative to the baseline (measured at week 0, 12, 24, 36, and 48), and the variations of total hippocampal atrophy and cerebrospinal fluid (CSF) Aβ (1-x), relative to the baseline (measured at week 24 and 48 in screening)

Safety variables were measured by abnormal reaction evaluation and a change in clinical trial results (general blood test, blood biochemical test, urinalysis).

According to the examples, it can be seen that PEP1 exhibits excellent effects on prevention and restoration of neuronal loss, and an increase in cognitive ability caused thereby in the experiments using animal models and the clinical trial, as well as at a cell level. Therefore, the peptide PEP1 according to the present invention may be developed as a drug for preventing or treating neuronal loss and a neurodegenerative disease caused thereby, and a fundamental preventive and therapeutic method for a neuronal disease using this drug may be provided. In addition, PEP1 is expected to show an excellent synergistic effect in co-treatment with a conventional therapeutic agent for a neurological disease.

## Claims

1. A composition for preventing and restoring neuronal loss, comprising:
one or more selected from a peptide including an amino acid sequence of SEQ ID NO: 1, a peptide having at least 80% sequence homology with the amino acid sequence and a fragment thereof at a pharmaceutically effective amount.

2. The composition of claim 1, wherein the fragment includes a fragment peptide consisting of three or more amino acids.

3. The composition of claim 1, wherein the composition is used to prevent, treat, alleviate and improve a disease caused by neuronal loss and regeneration inability, or to prevent, treat, alleviate and improve sequelae of the disease.

4. The composition of claim 3, wherein the disease caused by neuronal loss and regeneration inability is a cerebrovascular disease or a neurodegenerative disease.

5. The composition of claim 4, wherein the cerebrovascular disease is an ischemic cerebrovascular disease or a hemorrhagic cerebrovascular disease.

6. The composition of claim 4, wherein the neurodegenerative disease is any one of cerebropathia, spinal cord injuries, peripheral nerve damage, a peripheral nerve disease, amyotrophic lateral sclerosis, dementia, Huntington's disease, Parkinson's disease, an AD-like disease, spinocerebellar degeneration, and polyneuropathy.

7. The composition of claim 1, wherein the composition is co-treated with one or more therapeutic agents for a neurological disease.

8. The composition of claim 6, wherein the AD-like disease shows any one of abnormal symptoms including dementia, cognitive disorders and memory loss.

9. The composition of claim 6, wherein the AD-like disease is an early, progressive or severe AD-like disease.

10. The composition of claim 1, wherein the peptide of SEQ ID NO: 1 is administered as a pharmaceutically active ingredient at a dose of 0.0001 to 100 mg/kg/day.

11. The composition of claim 1, wherein the peptide of SEQ ID NO: 1 is administered as a pharmaceutically active ingredient at a dose of 0.01 to 200 mg/kg/day.

12. The composition of claim 1, wherein the peptide of SEQ ID NO: 1 is administered as a pharmaceutically active ingredient once every one week to every four weeks for a total of four or more times.

13. The composition of claim 1, wherein the composition is administered to inject the peptide of SEQ ID NO: 1 as a pharmaceutically active ingredient orally, intrarectally, percutaneously, intravenously, intramuscularly, intraperitoneally, intramedullarily, intrathecally, intracutaneously or subcutaneously.

14. A method for preventing and restoring neuronal loss, comprising:
administering a pharmaceutically effective amount of the composition of any one of claims 1 to 13 to a subject in need thereof.

15. A kit for preventing and restoring neuronal loss, comprising:
the composition for preventing and restoring neuronal loss according to any one of claims 1 to 13; and
an instruction of administering the peptide of SEQ ID NO: 1 as a pharmaceutically active ingredient to a subject in need thereof at 0.01 to 200 mg once every one week to every four weeks.


<110>     KIM, sangjae
          Gemvax & KAEL Co.,LTD.

<120>     A Peptide Having Neuronal Loss Inhibition and Neuron Regeneration
          Activity and the Composition Comprising the Same

<130>     OF16P242/PCT

<150>     KR 10-2015-0153518
<151>     2015-11-03

<160>     2

<170>     KopatentIn 2.0

<210>     1
<211>     16
<212>     PRT
<213>     homo sapiens

<400>     1
Glu Ala Arg Pro Ala Leu Leu Thr Ser Arg Leu Arg Phe Ile Pro Lys
  1               5                  10                  15

<210>     2
<211>     1132
<212>     PRT
<213>     homo sapiens

<400>     2